# EUROPEAN PATENT SPECIFICATION

(11) **EP 0 668 757 B1**
(45) Date of publication and mention of the grant of the patent: **10.12.2003**
(21) Application number: 94900613.4
(22) Date of filing: 11.11.1993
(51) Int. Cl.: A61K 9/107, A01N 25/04, A01N 25/10

(54) **COMPOSITIONS OF INSOLUBLE FILM-FORMING POLYMERS AND USES THEREFOR**
ZUSAMMENSETZUNGEN AUS UNLÖSLICHEN FILMBILDENDEN POLYMEREN UND IHRE VERWENDUNG
COMPOSITIONS DE POLYMERES FILMOGENES INSOLUBLES ET LEURS UTILISATIONS

(30) Priority: 13.11.1992 US 975811; 12.02.1993 US 17093
(43) Date of publication of application: 30.08.1995
(73) Proprietor: ISP INVESTMENTS INC., Wilmington, Delaware 19801 (US)
(72) Inventor: Narayanan, Kolazi S., Wayne,NJ 07470 (US)
(74) Representative: Ford, Michael Frederick
(86) International application number: PCT/US93/10881
(87) International publication number: WO 94/010979

(56) References cited:
- WO-A-91/08665
- WO-A-92/13454
- US-A- 4 482 534
- US-A- 4 544 693
- US-A- 5 061 751
- US-A- 5 093 031

## Description

### BACKGROUND OF THE INVENTION

A significant problem in the use of agriculturally active chemicals is that since they are applied to soil and plant surfaces, they are susceptible to being washed off by rain and/or water spray used for irrigation. This adversely affects the efficience of the chemicals, since the longer the chemical remains in contact with the plant or soil surface, the more effective it is.

Certain types of polymers exhibit film-forming properties and when dissolved in an organic solvent, can be applied for the purpose of providing a water-resistant coating on a substrate. Usually, the film-forming polymer in the solvent is applied to the particular substrate to be coated, and the solvent is allowed to evaporate or removed leaving a film of the polymer.

Generally, however, such water-resistant, film-forming polymers are soluble only in organic solvents, i. e., they are substantially insoluble in water. The use of such organic solvents generally is undesirable since they exhibit environmentally adverse properties, are often hazardous or flammable, and are generally expensive. In order to avoid the environmentally adverse effects of such organic solvents as well as to reduce the cost involved with using such solvents, rather complicated solvent recovery procedures must be used.

Typical of such polymers are copolymers of N-vinylpyrrolidone with α-olefins, vinyl acetate, styrene, acrylates, acrylic acids, amides, maleic acid, mono and diesters of maleic acids, and the like.

WO 92/13454 is concerned with concentrates containing agriculturally active chemicals which can be diluted to a microemulsion. The document discloses compositions containing a surfactant and also a lactam which may be an alkyl pyrrolidone. Some examples include a polymer which is polyvinyl pyrrolidone or a polyvinyl pyrrolidone/vinyl acetate co-polymer.

### SUMMARY OF THE INVENTION

The inventor has discovered a method for providing a stable microemulsion of a particular class of water-insoluble film-forming polymers in water. The microemulsion thus formed can be utilised to produce films of the particular film-forming polymer on a given substrate. For example, the microemulsion can be used as a coating for substrates, such as, wood, metal, glass. In addition, various active ingredients, e.g. fungicides, preservatives, insecticides, insect repellents, pheromones, radiation absorbents, dyes, can be included in the composition.

This invention provides an emulsion concentrate comprising:
a) 2 to 90% by weight of a solvent selected from the group consisting of long-chain alkyl pyrrolidones, synthetic or naturally occurring hydrophobic oils, and mixtures thereof;
b) 1 to 60% by weight of a water insoluble graft polymer of N-vinyl pyrrolidone and an alpha-olefin wherein the N-vinyl pyrrolidone is present in at least 20% on a weight basis; and
c) 1 to 85% by weight of a surfactant.

The amounts of the polymer surfactant and long-chain alkylpyrrolidone can vary within the broad ranges above. However, the relative compositional ranges of each must be such that a clear, stable microemulsion or solution of the insoluble polymer is obtained on the addition of water.

The weight percent of N-vinyl pyrrolidone is at least 20 percent or more but it may be higher, even at least 50% or more. The alpha-olefin may contain from 6 or 8 up to 20 or 30 carbon atoms, and preferably, from 16 to 20 carbon atoms. This copolymer may suitably have a number average molecular weight up to 50,000, e.g. 3,000 to 50,000.

The emulsion concentrate of the present invention provides a stable emulsion upon dilution with water. Thus, the emulsion concentrate of the invention is particularly suitable for use with an agriculturally active chemical or ingredient (hereinafter, sometimes referred to as a.i.).

The diluted inventive concentrate of the film-forming polymer may include an agriculturally active chemical or ingredient, such as, pesticides, herbicides, and the like. This composition may then be applied to plants, or soil, in the usual manner. The inventive composition, thus used, forms a film incorporating the a.i. on the leaf, soil or seeds and can prevent wash-out of the agriculturally active ingredient due to rain. Thus, for example, the composition with an agriculturally active ingredient forms a film on the particular substrate, e.g., the plant or soil, which results in improved retention and enhanced bioactivity of the agriculturally active ingredient and also provides superior rainfastness for such ingredients on leaf and substrate surfaces.

Notable agriculturally active chemicals include the isopropylamine salt of phosphonomethylglycine, pendimethalin, and 1,1-dimethyl-4,4'-bipyridinium dichloride.

### DETAILED DESCRIPTION OF THE INVENTION

As used herein, the following terms have the meaning indicated:
"macroemulsion" means an emulsion of water-in-oil or oil-in-water wherein the interior phase is in the form of visually discernable droplets and the overall emulsion is cloudy, and wherein the droplet diameter is greater than about 10 millimicrons (1 micron), and usually greater than 1000 millimicrons (about 10 microns).
"microemulsion" means an oil-in-water or water-in-oil, transparent thermodynamically stable dispersion of two or more immiscible liquids or a solid in a liquid wherein the dispersed phase consists of small droplets with diameters in the range of about 10 to 100 millimicrons. Such microemulsions are clear and appear as a single phase to the naked eye.
"single phase" as applied to a liquid means that to the naked eye, the liquid is homogeneous and does not appear to contain any other separatable liquid phase.
"clear" or "transparent" as applied to a liquid means that the liquid appears as a single phase without any particulate or colloidal material or a second phase being present when viewed by the naked eye.
"substantially insoluble" or "insoluble" means that for all practical purposes, the solubility of the compound in water is insufficient to make the compound practicably usable in an agricultural end use without some modification either to increase its solubility or dispersability in water, so as to increase the compound's bioavailability or avoid the use of excessively large volumes of solvent.
"High degree of loading in the concentrate" means an agriculturally active ingredient content of at least about 5 percent by weight.
"agriculturally active chemical or ingredient" (AAC or a.i.) means compounds and mixtures thereof which can be used as agricultural fertilizers, nutrients, plant growth accelerants, herbicides, plant growth controlling chemicals, and chemicals which are effective in killing plants, insects, microorganisms, fungi, bacteria and the like which are commonly referred to as insecticides, bactericides, fungicides, nematocides, fumigants, synergists, i.e., compounds which when used in conjunction with other AAC's enhance their activity and the like, as well as any other chemicals having properties which are suitable for agricultural uses in terms of application to plants or domestic uses for controlling insects and pests.

Long-chain N-alkyl pyrrolidones suitable for use as a solvent in the present invention have the formula 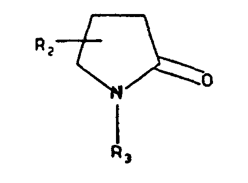 wherein R₂ is hydrogen or alkyl having from 6 to 14 carbon atoms and R₃ is alkyl having from 6 to 14 carbon atoms with the proviso that at least one of R₂ and R₃ must contain at least 6 carbon atoms and the sum of the carbon atoms in R₂ and R₃ cannot exceed 14. Preferably, R₂ is hydrogen and R₃ is C₈ or C₁₂. Mixtures of two long-chain alkyl pyrrolidones may also be used. N-methyl pyrrolidone may also be included along with long chain N-alkyl pyrrolidones in an amount effective to help maintain the solubility of the long chain alkyl pyrrolidones.

Notably, the N-octyl pyrrolidone may be used jointly with N-dodecyl pyrrolidone and N-methyl pyrrolidone in proportions of 0.1 to 10 parts, especially 0.5 to 2 parts each of N-dodecyl pyrrolidone and N-methyl pyrrolidone to 1 part of N-octyl pyrrolidone.

The solvent may be an organic diluent which is a synthetic or naturally occurring oil having a high hydrophobic character or having a fractional dispersive solubility parameter of greater than 70% and preferably greater than 85% and a molar volume of greater than 90 cm³/mole. These properties are defined in the C.R.C. Handbook referred to hereinabove. Typical diluents include soybean oil, rapeseed oil, long chain alcohols, long chain ketones, long chain esters, and ethers. As used herein, "long chain" means with 6 or more carbon atoms. Also suitable as the organic diluent are aromatic petroleum oils including those which are commercially available distillates from crude oils having an average boiling point greater than 120°C. Typical of such materials are those sold under the trademarks Exxon 200 or Texaco 400. Of course, such aromatics should be approved for use as a carrier for agriculturally active chemicals.

The composition of the aromatic petroleum oil is generally:
Heavy aromatic solvent naphtha - about 60%;
Middle distillate solvent extractant - about 40%;

Normally, these oils contain predominantly the C9-C15 aromatic hydrocarbons and primarily the C10-C12 hydrocarbons having a flash point of about 95°C (203°F).

Suitable surfactants include both water and oil-soluble surfactants, e.g., ethoxylated alkyl phenols, linear aliphatic polyesters, linear aromatic polyesters, polyalkenyloxyalcohol, linear aliphatic ethoxylates, polyethoxylated castor oil, polyethoxylated carboxylates, and polyethoxylated alkylamines. Oil and water-soluble anionic surfactants may be used as the emulsifier and include phosphate esters and their salts, alkyl sulfates, sulfonates, and their salts, salts of sulfate nonylphenoxypoly(ethyleneoxy) ethanol, salts of alkylbenzene sulfonates, e.g., the sodium, calcium and alkylammonium salts, salts of alkylnaphthalene sulfonate, and sulfonated aliphatic polyesters and their salts. Also suitable are complex phosphate esters of nonionic surfactants of the ethylene oxide type which are mixtures of diesters of phosphonic acid. (See, for example, McCutcheon's, Emulsifiers and detergents (1989), published by McCutcheon's Division of M.C. Publishing Co., Glen Rock, New Jersey.)
Polymers particularly suitable for use in the present invention include polymers, such as, Ganex 516, which is copolymer of an alpha-olefin and N-vinyl pyrrolidone (50/50) percent mixture, copolymers of vinyl pyrrolidone and alpha-olefins. Typically, such alpha-olefins contain up to 20 carbon atoms, and preferably contain 16 carbon atoms. The weight average molecular weight of such polymers is generally greater than about 10,000. One specific possibility is a graft copolymer of N-vinyl pyrrolidone and an alpha-olefin containing 16 carbon atoms in a weight ratio of 50:50 and having a number average molecular weight of about 9500. Another is a copolymer of N-vinyl pyrrolidone and C₂₀ alpha-olefin in a 20:80 weight ratio. Particularly suitable are water- insoluble polymers, such as, Agrimer AL25 (International Specialty Products (ISP) Corporation), which is a copolymer of an alpha-olefin having the formula C₁₄H₂₉CH=CH₂ (50%) and N-vinyl pyrrolidone (50%), and Agrimer AL30 (ISP Corporation), which is a copolymer of an alpha-olefin having 20 carbon atoms (80%), and N-vinyl pyrrolidone (20%).

The inventive concentrate comprises from 2 to 90 percent, preferably from 5 to 85 percent, and most preferably, from 30 to 80 percent by weight of N-alkyl pyrrolidone, organic solvent oil or both; from about 2 to preferably 30 and most preferably, from 8 to 15 percent by weight surfactant, and from 1 to 60, and preferably from about 5 to 30 percent, and most preferably, 10 to 25 weight percent of the water insoluble polymer. Water may be present in an amount from 1 to 85 % and, preferably, from 3 to 70 percent, and most preferably, from about 5 to 60 percent by weight.

All percents herein are percent by weight based on the total weight of the composition.

The inventive compositions are particularly suitable for end use applications wherein films of water-insoluble polymers are formed on substrates. The films may be formed for adhesive, protective, decorative or lubricating purposes, or to impart hydrophobicity or hydrophilicity. Since it is desirable to avoid organic solvents due to their cost and adverse toxicological and environmental properties, the use of water as a solvent for the film-making procedure is preferred. With the inventive composition, it becomes possible to place such ordinarily water-insoluble film-forming polymers in an aqueous based vehicle, i.e., solution, emulsion or dispersion-solubilizing liquid, which can be handled and utilized in the same manner as a true solution of the polymer to form a film therewith. Thus, the inventive composition in microemulsion form may be coated as is, or after further dilution with water, if desired, onto a substrate. The water is then removed as by evaporation to leave the polymer film remaining.

The inventor has further discovered that the rainfastness of agriculturally active ingredients, and in particular, pesticides, can be substantially improved by fonnulating the pesticides in the inventive composition including the water insoluble film-forming polymer. Many pesticides, and particularly water soluble agriculturally active chemicals, are washed off by rain after they have been applied to the plants or soil. This also applies to herbicides. For effective pest and weed control, it takes from a few hours to three weeks for the pesticide or herbicide to penetrate into the biological system. The present invention assures that the agriculturally active ingredient will be retained for a sufficiently long time to allow it to be effective and avoid or reduce rain wash-off.

In use, the inventive composition is diluted with water and applied to the crop, plants, or soil. Normally, this dilution is carried out at the field site. As used herein rainfast resistant, rainfast or rainfastness in connection with the inventive compositions means that a film formed from the composition exhibits increased resistance to removal by water washing as compared to the same composition which does not contain the film-forming polymer under the test procedures as described hereinafter.

Pesticides which can be used with the present invention, may be characterized by their physical properties, depending on their physical state at normal or ambient conditions, i.e., between 4.4°C and 32.2°C (40°F and 90°F) and their solubility or miscibility with water or other common organic solvents, e.g., aromatics, such as, toluene, xylene, methylated and polyalkylated naphthalenes, and aliphatic solvents.

Based on the physical properties, the pesticides may be classified into three groups:
The first group includes those which are oily liquids at ambient temperatures and are immiscible with water. Specific pesticides include:
   Common esters of 2,4-dichlorophenoxyacetic acid,
   Common esters of 2,4,5-trichlorophenoxyacetic acid,
   Common esters of 2-(2,4-dichlorophenoxy) propionic acid,
   Common esters of 2-(2,4,5-trichlorophenoxy) propionic acid,
   Common esters of 2,4-dichlorobutyric acid,
   Common esters of 2,methoxy-3,6-dichlorobenzoic acid,
   Common esters of 2-methyl-4-chlorophenoxyacetic acid,
   Piperonyl butoxide 3,4-methylenedioxy-6-propyl benzyl n-butyl diethylene glycol ether,
   Bromophos ethyl: 0,0-diethyl-0-2,5-dichloro-4-bromophenyl thionophosphate, N-(2-mercaptoethyl) benzene-sulfonamide (BETASAN®),
   Isobornyl Thiocyanoacetate (THANITE®),
   Ioxynil ester of octanoic acid,
   Molinate S-ethyl hexahydro - 1 H - azepine-1-carbothioate,
   PP 511 0,0-dimethyl-(2-diethylamine 4-methyl-6-pyrimidinyl) carbamate,
   PP 211 0,0-diethyl O-(2-diethylamine-4-methyl-6- pyrimidinyl) phosphorocarbamate, 5-Ethoxy-3-(trichlorometyl)-1,2,4-thiadiazole (TERRAZALE®),
   Ethyl-s-s-dipropyl-phosphodithioate (MOCAP®),
   S-Ethyl dipropylthiocarbamate (EPTAM®),
   S-Ethyl diisobutylthiocarbamate (SUTAN®),
   S-n. propyl-di-n-propylthiocarbamate (VERNAM®),
   S-propyl butylethylthiocarbamatae (TILLAM®),
   S-ethyl ethylcyclohexylthiocarbamate (RO-NEET®),
   Malathion (S-(1,2-dicarboxyethyl)-O,O-dimethyl phosphorodithioate),
   Diazinon (0,0-diethyl,0-(2-isopropyl-4-methyl-6-pyrimidinyl) phosphorothioate, O-Ethyl-S-phenyl-ethylphosphonodithioate (DYFONATE®),
   Toxaphene (Octachlorocamphene),
   Bromoxynil (3,5-dibromo-4-hydroxy benzonitrile ester of n.octanoic acid, 2-chloro-N-2,6-diethylphenyl-N-methoxymethylacetamide (LASSO®),
   Diallate S-2,3-dichloroallyl N,N-diisopropylthiolcarbamate,
   Triallate S-2,33-trichloroallyl N,N-diisopropylthiolcarbamate.

The second group comprises those pesticides which are solids at ambient temperatures and for all practical purposes, insoluble in water.
2,4,5-T (2,4,5-trichlorophenoxy acetic acid)
Monuron (3-(p-chlorophenyl)-1,1-dimethyl urea)
Diuron (3-(3,4,-dichlorophenyl)-1,1-dimethyl urea)
Bromacil (5 bromo-3-sec. butyl-6-methyl uracil)
Isocil (5 bromo-3-isopropyl-6-methyl uracil)
Linuron (3-(3,4 dichlorophenyl)-1-methoxy-1 methyl urea
Atrazine (2-chloro-4-ethylamino-6 isopropylamino-s- triazine) Simazine (2-chloro-4,6,-bis (ethylamino)-s-triazine
Dodine (n-dodecylguanidine acetate)
Thiram (tetramethylthiuram disulfide)
N-(mercaptomethyl)phthalimide s-(o,o dimethylphosphorodithioate) (IMIDAN®) Lindane (gamma 1,2,3,4,5,6 hexachlorocyclohexane)
Folpet (N-trichloromethylphthalimide)
Manazon (s-(4,6-diamino-1,3,5-triazin-2-yl methyl)dimethyl phosphorothiolthionate)
Barban (4-chloro-2 butynyl m-chlorocarbanilate)
Tricumba 2-methoxy-3,5,6-trichlorobenzoic acid
Trifluralin (2,6-dinitro-N,N-dipropyl-4-trifluoromethylaniline) (2,3 dihydro-5-carboxanilido-6-methyl-1,4-oxathiin) (VITAVAX®)
2,4-dichlorophenoxyacetic acid
4-(4-chloro-2 methylphenoxy) butyric acid
2-(2,4-dichlorophenoxy) propionic acid
Ioxynil: 3,5 diiodo-4-hydroxybenzonitrile
Bromoxynil: 3,5 dibromo-4-hydroxybenzonitrile
Methoxychlor: 2,2,-Bis(p-methoxyphenyl)-1,1-trichloroethane
PP 781: 4(2-chloro phenylhydrazono)-3-methyl-5-isoxazolone*
PP 675: 5-butyl-2-dimethylamino-4-hydroxy-6-methyl pyrimidine*
PP 062: 5,6-dimethyl-2-dimethylamino-4 pyrimidinyl dimethylcarbamate*
PP 149: 5-n-butyl-2 ethylamino-4-hydroxy-6 methylpyrimidine*
C 6313 N'-(4-bromo-3-chlorophenyl)-N-methoxy-N-methylurea
C 6989 2,4'dinitro-4-trifluoromethyl-diphenylether
Chloroxuron N'-4-(chlorophenoxy) phenyl-NN-dimethylurea
Dichlobenil 2,6-dichlorobenzonitrile
Diphenamid NN-dimethyl-2,2-diphenylacetamide
Fenac 2,3,6-trichlorophenylacetic acid
Fluometuron N'-(3-trifluoromethylphenyl)-NN-dimethylurea
GS 14260 4-ethylamino-2-methylthio-6-t-butyl-amino-1,3,5-triazine
PCP Pentachlorophenol
Lenacil 3-cyclohexyl-6,7-dihydro-1H-cyclo-pentapyrimidine-2,4-(3H,5H)-dione
Pyrazon 5-amino-4-chloro-2-phenyl-3-pyridazone
Metrobromuron N'-(4-bromophenyl)-N-methoxy-N-methylurea
Metoxymarc N-(4-methoxybenzoyl)-N-(3,4-dichlorophenyl)-N',N'-dimethylurea Neburon N-butyl-N'-(3,4-dichlorophenyl-N-methylurea
NIA 11092 1,1-dimethyl-3-[3-(n-t-butyl carbamyloxy)phenyl] urea
Mecoprop 2-(4-chloro-2 methylphenoxy)propionic acid
Monolinuron N'-(4-chlorophenyl)-N-methoxy-N-methylurea
Nitrofen 2,4-dichlorophenyl 4-nitrophenylether
Propanil N-(3,4-dichlororphenyl)propionamide
Pyriclor 2,3,5-trichloro-4-pyridinol
Solan 3'-chloro-2-methyl-p-valerotoluidide
Terbacil 5-chloro-3-t-butyl-6-methyluracil
UC 22463 (SIRMATE)-3,4-dichlorobenryl N-methylcarbamate
WL 9385 2-Azido-4-ethylamino-6-t-butylamino-s-triazine
Propachlor 2-chloro-N-isopropylacetanilide
CP 50144 2-chloro-N-2,6-diethylphenyl-N- methoxymethylacetamide
CP 31675 2-chloro-N-(2 methyl-6-t-butylphenyl)acetamide
Cypromid 3',4'-dichlorocyclopropane carboxanilide
Fenuron NN-dimethyl-N-phenylurea
Chlorbromuron N'-(4-bromo-3-chlorophenyl)-N-methoxy-N-methylurea
Ametryne 2-methylxnercapto-4-ethylamino-6-isopropyl-amino-s-triazine
Prometryne 2-methylmercapto-4,6-bisisopropyl amino-s-triazine
DCPA dimethyl 2,3,5,6, tetrachloroterephthalate
Benefm N-butyl-N-ethyl-2,2,2-trifluoro-2,6-dinitro-p-toluidine
Nitralin 2,6-dinitro-4-methylsulfonyl-N, N-dipropyl-aniline
PP 493 2,6-difluoro-3,5-dichloro-4-hydroxy pyridine
CNP 2,4,6-trichlorophenyl-4'-nitrophenyl ether
Pentachloro nitrobenzene
1-(butyl carbamoyl)-2-benzimidazole carbamic acid, methyl ester (BENLATE®). Carbaryl (methylnaphthyl carbamate) (active ingredient in Sevin).

The third group constitutes those compounds which are water-soluble, such as, salts, e.g., the isopropylamine salt of phosphonomethyl glycine, the sodium salt of 2,4-dichlorophenoxy acetic acid, the sodium salt of methoxy dichloro benzoic acid (dichloro anisic acid), and dicamba (dimethylamine salt of methoxy dichlorobenzoic acid), Assert bisulfate (American Cyanamid), the ammonium salt of imazaquin (American Cyanamid).

The following Examples illustrate the invention:

The materials used in the Examples and designated by trademark or tradename are as follows:
- Agrimer AL25: copolymer of vinyl pyrrolidone and C₁₆ α- olefm in 50:50 weight ratio with a number average molecular weight of about 9500;
- Agrimer AL30: graft copolymer containing 80% by weight of C₂₀ α-olefin and 20% by weight of polymerized vinylpyrrolidone with a number average molecular weight of about 8600 available as a solid;
- Agrimer AL22: graft copolymer containing 80% by weight of C₁₆ α-olefin and 20% by weight of polymerized vinylpyrrolidone with a number average molecular weight of about 7300 available as a liquid;
- Agrimer VA3: copolymer containing 30 mole % vinylpyrrolidone units and 70 mole % vinylacetate unit with a number average molecular weight of 5700 and weight average molecular weight of 28,800 determined by the GPC method;

- Acrylidone ACP-1004: copolymer containing 50:50 weight ratio of the monomer with number average molecular weight = 30,000 - 60,000, 100,00 - 300,000.
- Rodeo: a commercially available pesticide of an aqueous solution containing 53.8% of the isopropylamine salt of phosphonomethylglycine (a.i.-1) (Monsanto).
- Latron B1956: 77% modified phthalic glycol alkyl resin, and 23% inert ingredients including organic solvent (Rohm & Haas);
- Prowl: an emulsion concentrate containing 42.0% by wt. pendimethalin (a.i.-2). The balance constitutes surfactants and solvents.
- Roundup: a commercially available concentrate of glyphosate with suitable wetting agents. Glyphosate is the isopropylamine salt of phosphonomethylglycine.
- Gramoxone: a commercially available concentrate of Paraquat in suitable wetting agents. Paraquat is 1,1-dimethyl-4,4'-bipyridinium dichloride (American Cyanamid).
- Alcasurf CA: a commercial form of calcium dodecyl benzene sulfonate produced by Rhone Poulenc containing an oil-soluble anionic surfactant (0.32 parts propylene glycol, 0.65 parts n-butanol, 3.90 parts calcium dodecyl benzene sulfonate and 1.62 parts of aromatic hydrocarbons).
- Pendimethalin: N-(1-ethylpropyl)-3,4-dimethyl-2,6-dinitrobenzenamine.

- Fusilade EC: a commercial formulation of the active ingredient butyl 2-[4-(5-trifluoromethyl-2-pyridyloxy)phenoxy] propionate (FUSILADE 2000) which is a selective systemic herbicide for control of grass weeds in broadleaved crops.
- Thiadiazuron: N-phenyl-N'-1,2,4-thiadiazol-5-yl urea.

### Example 1

Compositions containing water insoluble polymers in a water-based medium were prepared as follows:

### Preparation of Matrix

In a 113 g (4 ounce) stoppered glass bottle, 48.33 g of N-octylpyrrolidone, and 41.67 g of a 29% by weight aqueous solution of sodium dodecyl sulfate (commercially available) were added and stirred to form a homogeneous solution containing 48.33 g of N-octylpyrrolidone, 12.06 g of sodium dodecyl sulfate and 29.61 g water. This solution is referred to herein as the stock matrix solution. To 90 g of the stock matrix solution were added 10 g of the dry water-insoluble Agrimer AL25. The solution was stirred in an automatic orbital shaker at 400 RPM for a period of 3 hours, which produced a homogeneous single phase system. On 10X, 500X and 1000X dilution with deionized water, the diluted composition remained homogeneous for more than 72 hours. The dry Agrimer AL25 was prepared by evaporation of the commercially available 50 percent solution of AL25 in isopropyl alcohol under reduced pressure, drying the residue in vacuum oven at about 60 - 70°C at 1 mm Hg., and then grinding under a blanket of liquid nitrogen to produce a particulate solid.

### Example 2

Example 1 was repeated using 85 parts of stock matrix solution and 15 parts of the polymer. The resulting composition is shown in Table 1 and is referred to herein as Composition I. This composition was also in a homogeneous single phase system. On 10X, 500X, and 1,000X dilution with deionized water, the diluted composition remained homogeneous for more than 72 hours.

### Example 3

Example 1 was repeated using 80 parts of stock matrix solution and 20 parts of the polymer. The resulting composition is shown in Table 1. At 10X and 500X dilution with deionized water, the compositions produced exhibited no separation for more than 72 hours. When 1 part of the composition was diluted to 1000 parts with deionized water, a single minute crystal was observed.

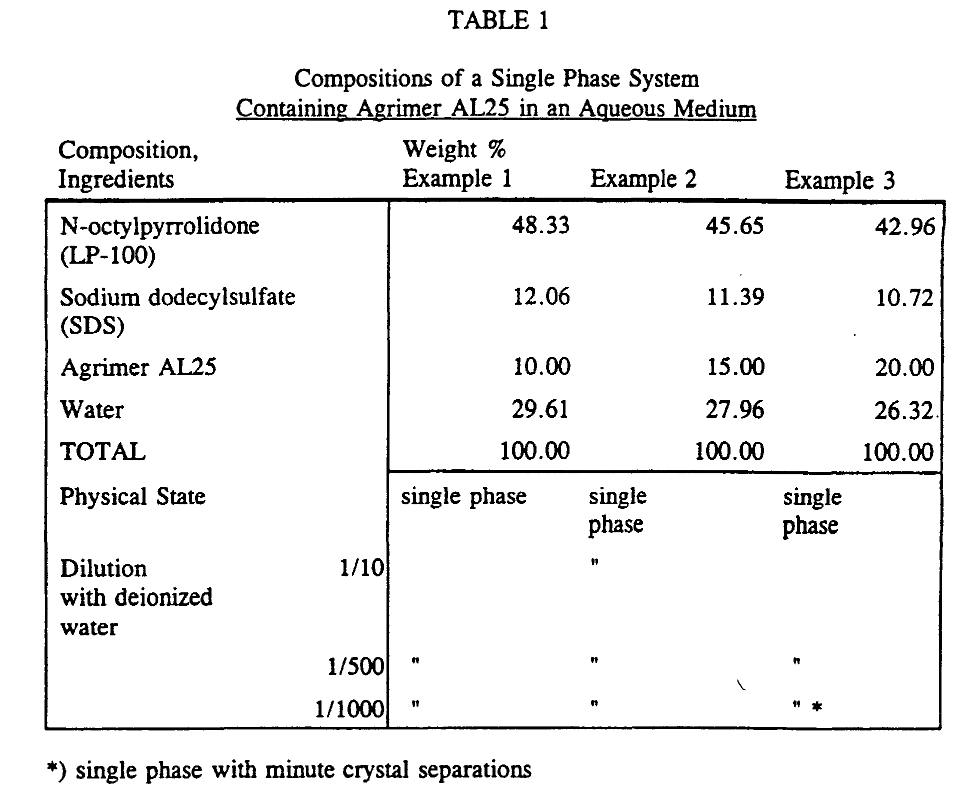

### Example 4

This example illustrates the inability of an isopropyl alcohol (IPA) solution of Agrimer AL25 to remain homogeneous in the absence of the inventive stock matrix. A composition was prepared by mixing 40 g of a 50 % solution of Agrimer AL25 in IPA, 35.72 g of a 29% aqueous SDS solution and 24.20 g of deionized water. The resulting composition contained by weight: 25% Agrimer AL25, 20% IPA, 10.36% SDS, and 49.64% water. The resulting product was a milky, cloudy emulsion. Microscopic observation at 250x showed particles/oil droplets of about 5-10 µ in size.

Examples 5 and 6 illustrate the advantage of the presence of more than 20% on a weight basis of vinyl pyrrolidone in the copolymer. Examples 7 and 8 are outside the invention.

### Example 5

90 parts stock matrix solution, and 10 parts of Agrimer AL30 were mixed in an automatic orbital shaker for 4 hours. No dissolution occurred.

### Example 6

Example 5 was repeated replacing Agrimer AL30 with 10 g of Agrimer AL22. After stirring for 4 hours in an automatic orbital shaker, oil separation was observed.

### Example 7

Example 4 was repeated using 98 parts of stock matrix solution and 2 parts of Agrimer VA3. The composition did not produce a single phase system on stirring in an automatic orbital shaker for 4 hours.

### Example 8

Example 4 was repeated with 95 parts of stock matrix solution and 5 parts of Acrylidone ACP-1004. A homogeneous phase was not obtained after 4 hours of stirring.

Example 4 was also repeated using 95 parts of stock matrix solution and 5 parts of cross-linked polyvinylpyrrolidone with a molecular weight of about 1 MM. A single phase system was not obtained. However, the polymer was wetted by the matrix.

### Example 9

### Part A

A series of polymer films formed from the inventive composition were evaluated for rain-fastness. The formulations were prepared as follows:
1. A commercial formulation of a given agriculturally active ingredient was admixed with the inventive composition.
2. The liquid mixture was then diluted to end use concentrations.
3. An appropriate dose (0.1 g to 0.5 g) was applied to a 15 cm by 15 cm (6 inch by 6 inch) glass plate uniformly as a 2.5 to 7.5 cm (1 to 3 inch) square patch. The patch was dried in a hood under ambient conditions for 48 to 72 hours.
4. After a dry film was formed, a fine spray of water was applied to simulate 1.25 to 5 cm (0.5 to 2 inches) of rain wash off. The washings were collected in a waste jar.
5. The remaining washed patch was extracted with a solvent (ethanol is preferred) quantitatively into a 100 ml volumetric flask. If desired, appropriate dilutions of the ethanol extract were made.
6. The ethanol extract was subjected to ultraviolet spectra examination and the absorbance at a X max. was determined (value X₁).
7. Blank samples of diluted formulations were prepared and measured using UV absorption. These values were utilized to prepare a calibration chart. This value is X₂.
8. The percent retained was then determined by dividing X₁ by X₂ and multiplying by 100.
9. Blanks were run without polymer using the commercial formulations under identical conditions. The percent retained is identified as R_{b}.
10. For samples wherein the pesticide used is Pendimethalin, λ max. is 239 nm. 0.3 g of diluted samples were used for patches (6.45 to 12.9 cm², i.e. 1 to 2 square inches). The amount of wash-off water used was 1.5 to 2.5 g.

Using this technique, a series of compositions were formulated and tested for retention of the active ingredient.

### Part B

### Rainfast evaluations

### The following solutions were prepared:

Solution 9A
   4.0036 g of Rodeo was diluted to 100 g with deionized water. This solution contained 2.15% a.i.-1. on a weight basis.
Solution 9B
   4.0101 g Rodeo was mixed with 0.13 g of Composition I (see Example 2 and Table 1 above) and diluted to 100 g with deionized water producing a clear solution.
Solution 9C
   4.0033 g Rodeo was mixed with 0.34 g of Composition I and diluted to 100 g with deionized water producing a clear solution.
Solution 9C
   4.0095 g Rodeo was mixed with 0.66 g of Composition I and diluted to 100 g with deionized water producing a clear solution.

Samples of solutions 9A, 9B, 9C, and 9D were analyzed for the a.i.-1. content via potentiometric titration using standard NaOH (0.0217 N). The NaOH was standardized using a standard solution of sodium hydrogen phthalate. For 0.6911 g of 9A, 0.7044 g of 9B, and 0.7189 g of 9D, 3.00 ml of NaOH was required. For 0.7045 g of 9C, 2.95 ml of the NaOH solution was required for neutralization. The % a.i.-1. for the samples 9A, 9B, 9C, and 9D were 2.17, 2.13, 2.13, and 2.08%, respectively, against the theoretical value of 2.15 %, thus validating the experimental procedure.

### Rainwash Treatment

Samples of solutions 9A, 9B, 9C, and 9D were spotted on glass plates using the above-described procedure and dried at room temperature for 72 hours. The dried films were subjected to a fine spray of water using a spray bottle delivering a fine spray of water from an aspirator. The amount of water delivered was 1.49 g to 1.54 g. The remaining film was recovered into a beaker by washing the slide with a 10% EtOH aqueous solution and the washings were titrated with 0.0219 N NaOH. The endpoint of the titration was evaluated either potentiometrically or by using phenolphthalein as the indicator.

The % a.i.-1. retained was calculated by dividing the experimental concentration of a.i.-1 by the theoretical or actual concentration of the a.i.-1 found in the samples. Table 2 summarizes the results.

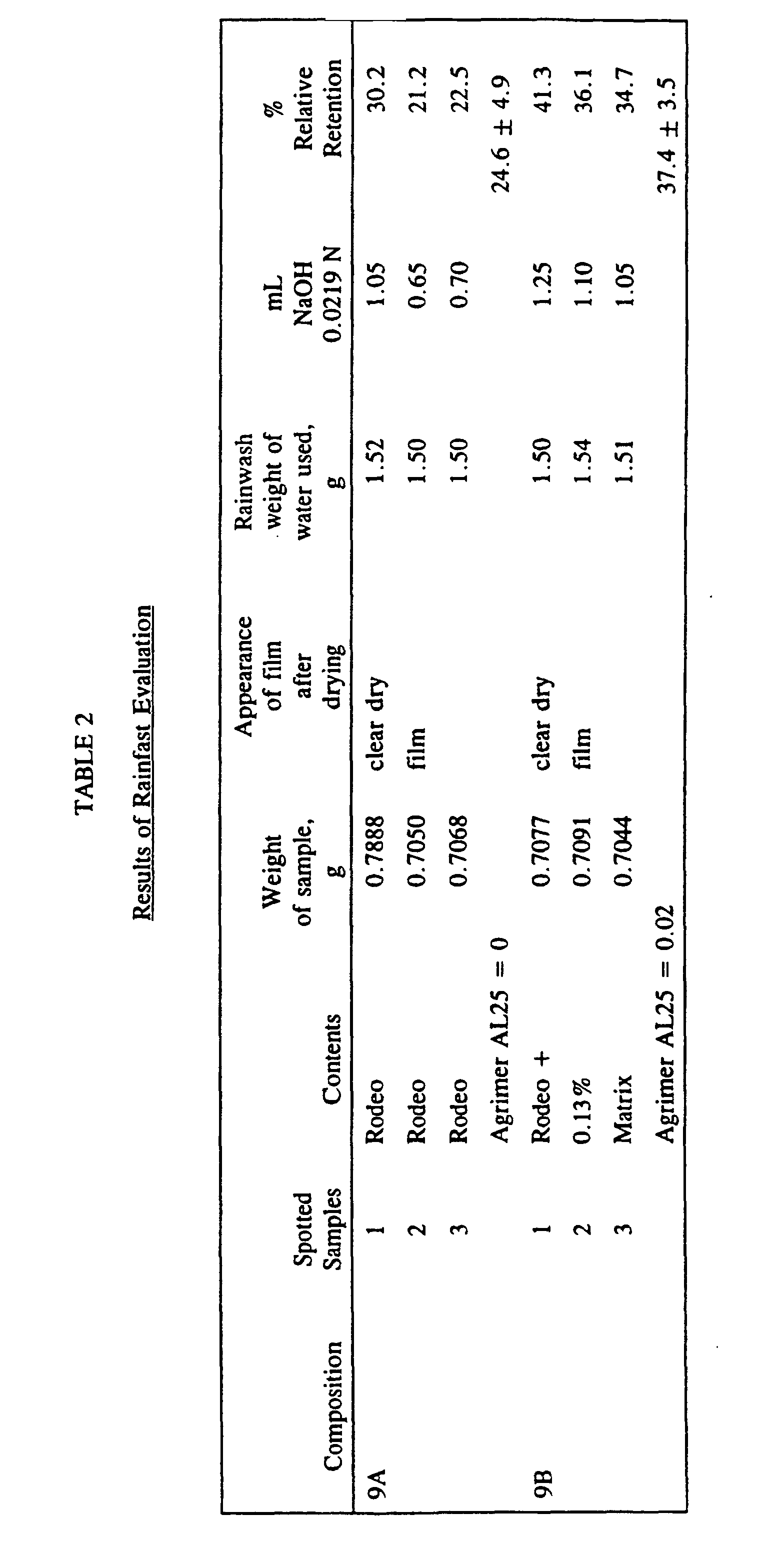

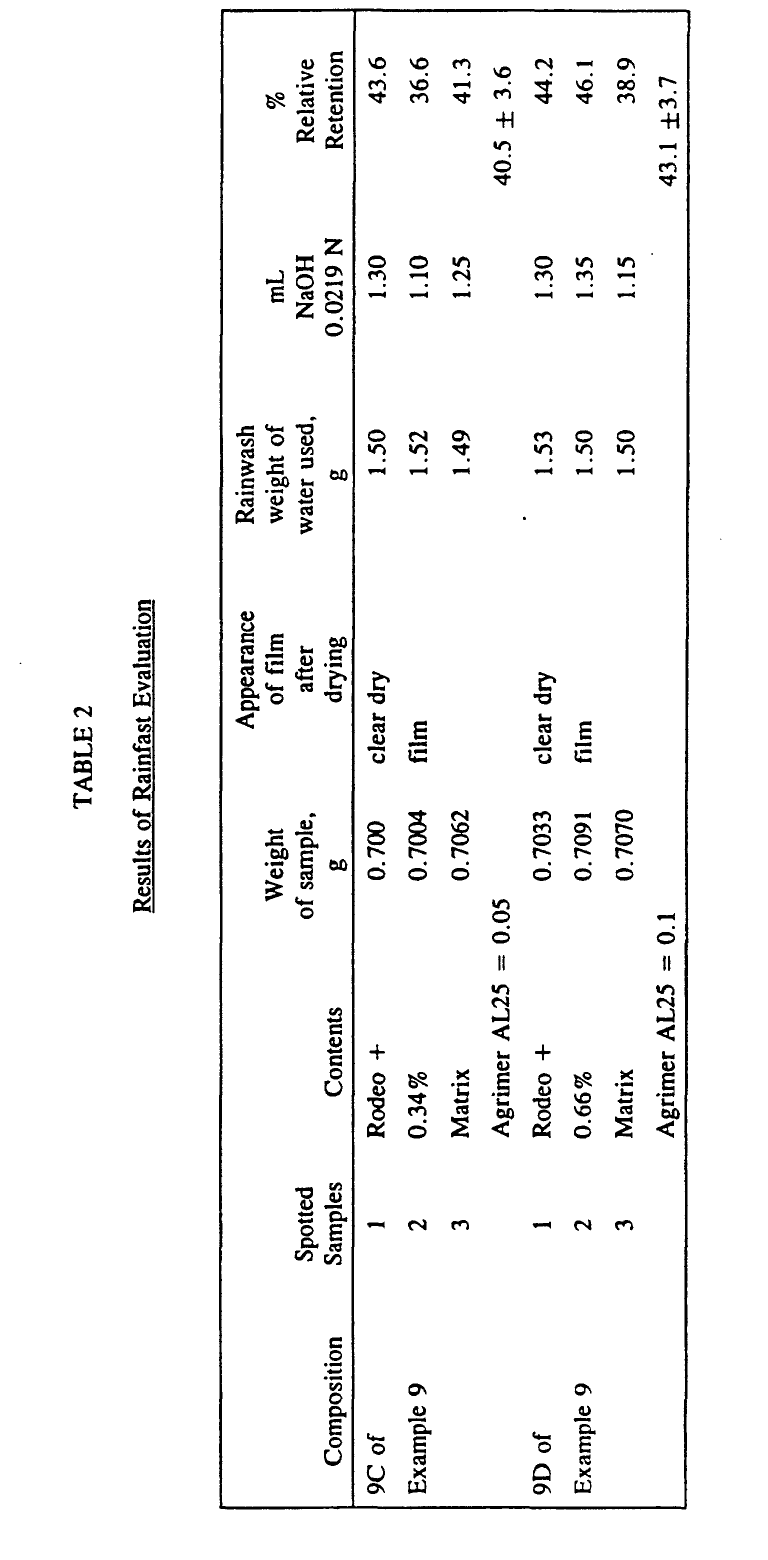

### Example 10

The procedure of Example 9 was repeated using the following solutions:
- 10A:: 4.0069 g Rodeo diluted to 100 g with deionized water;
- 10B:: 4.0018 g Rodeo and 0.1559 g of Composition I diluted to 100 g with deionized water;
- 10C:: 4.0076 g Rodeo and 0.1133 g of the stock matrix solution of Example 1.

The results are shown in Table 3.

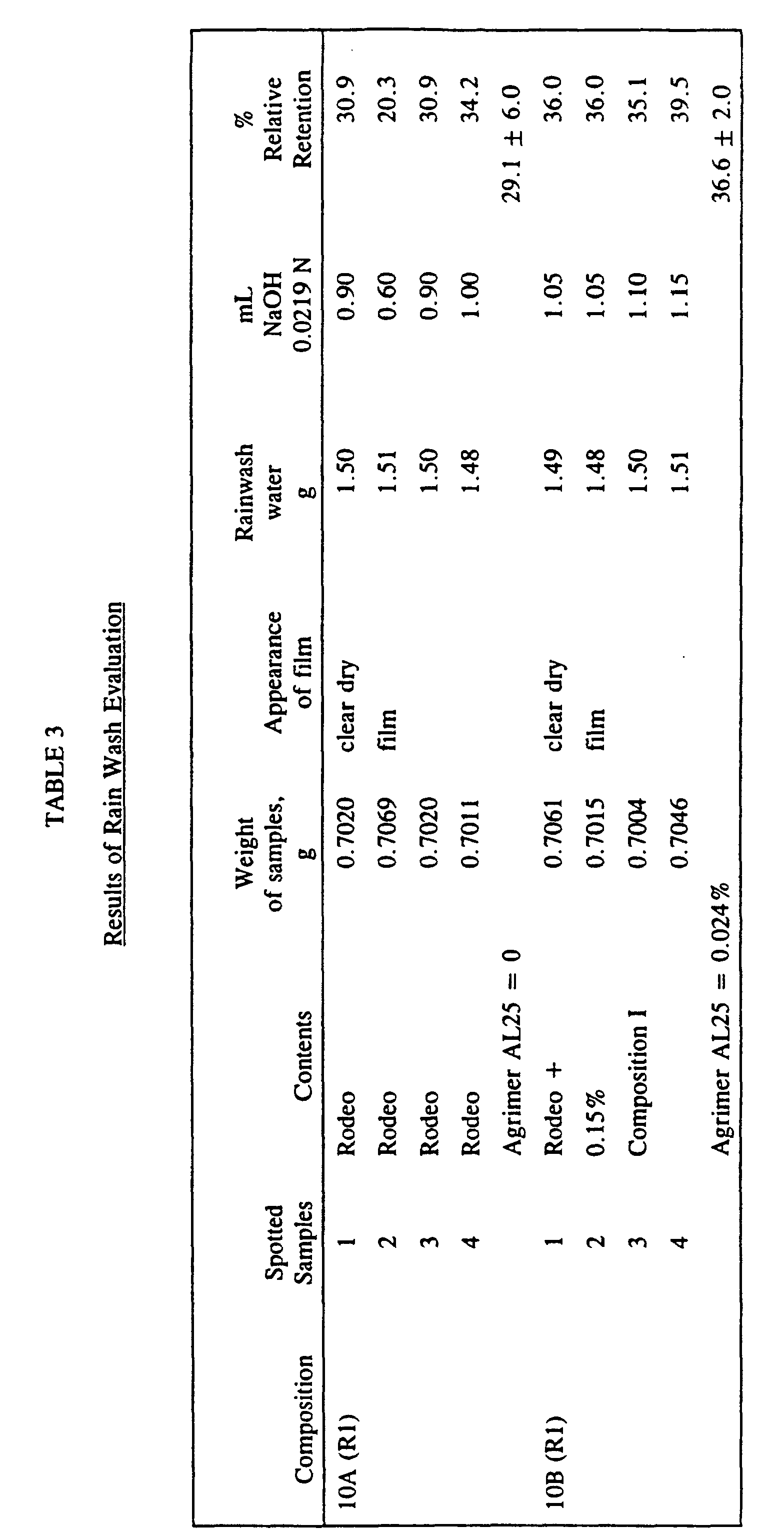

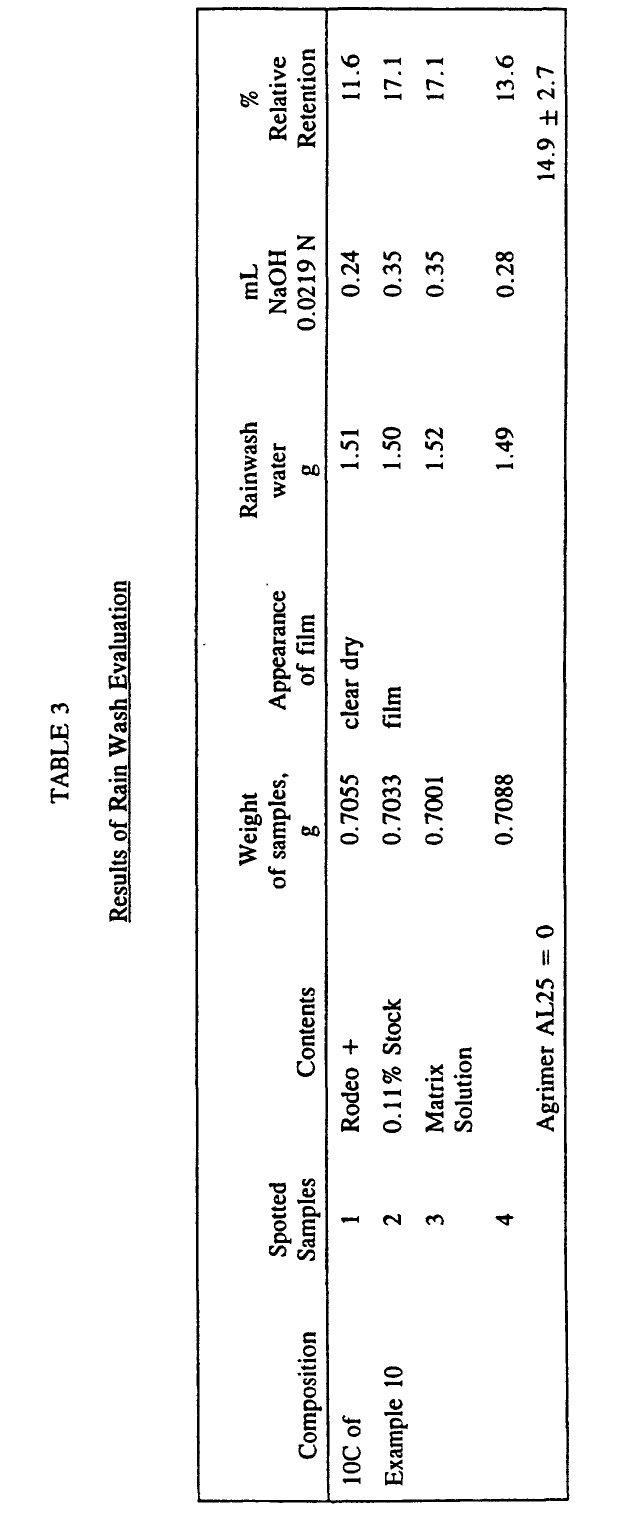

The difference between Examples 10B and 10C is the presence of polymer Agrimer AL25 which increases the retention of the water soluble a.i.-1. from 14.9% to 36.6%. The matrix when used alone without the polymer accelerates the washing of the a.i. The polymer forms a water resistant film when used in combination with the matrix and retains the a.i.-1.

### Example 11

### Evaluation of Rainfastness of Rodeo with and without Composition I

The procedure of Example 9 was repeated using compositions 9A and 9B (herein designated 11A and 11B, respectively). However, the glass plate was covered by parafilm sheets to provide a hydrophobic surface similar to that of a plant leaf surface. Composition 11A was prepared by diluting 4.000 g of Rodeo to 100 g with deionized water to form a homogeneous solution. Composition 11B was prepared by mixing 4.0101 g of Rodeo and 0.3327 g of Composition I and diluting the mixture to 100 g with deionized water. The results are shown in Table 4.

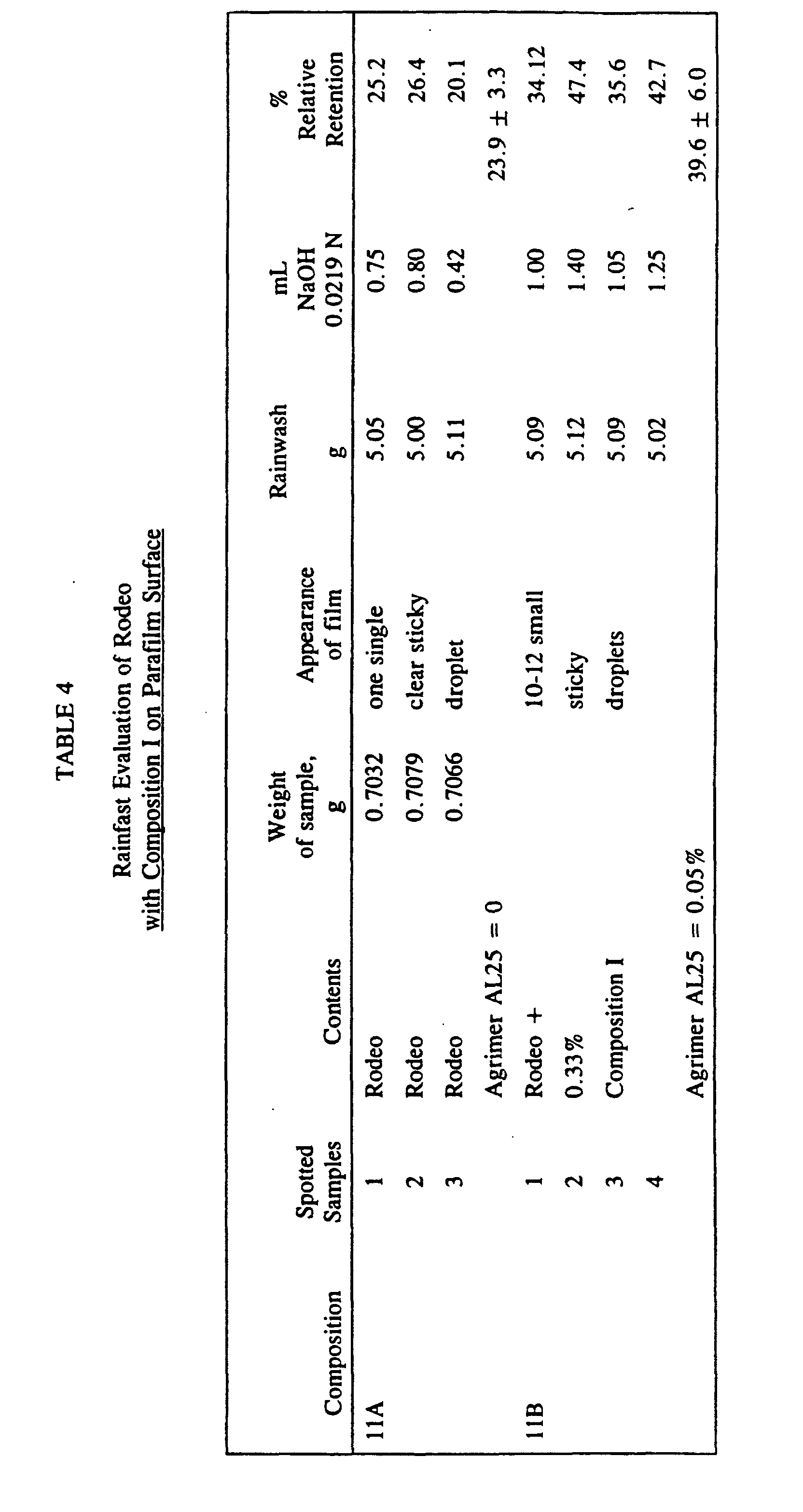

### Example 12

A rainfast evaluation of Rodeo with commercially available Latron B1956 as a tank mix additive was carried out. For comparison, samples of Latron B1956 stripped of its solvent and Composition I with Rodeo were prepared. The mixture was diluted before use after storing the concentrate with the adjuvant for 72 hours. The following compositions were prepared:
- 12A-R3:: 4.00 g Rodeo diluted to 102 g with deionized water to produce a clear homogeneous solution.
- 12A-R4:: 4.00 g Rodeo diluted to 100 g with deionized water.
- 12A:: 4.00 g Rodeo was mixed with 0.2 g Latron B1956 diluted to 100 g to produce an emulsion.
- 12B:: 100 g of commercial Latron was stripped of the volatile solvents by evaporation at 82° C. under 65 mm Hg vacuum for a period of 2 hours. The weight loss was 19 g. The resulting product (81 g) is designated solvent stripped Latron B1956. 1.57 g of the solvent stripped Latron was mixed with 29.26 g Rodeo and stored as a concentrate containing 95 % Rodeo and 5% a solvent stripped Latron B1956.

The concentrate described above was stored for 72 hours. 4.00 g was diluted to 100 g with deionized water to produce an emulsion.
- 12C:: 22.924 g Rodeo was mixed with 2.075 g of Composition I to produce a composition containing 91.69% Rodeo and 8.30% of Composition I. The mixture was an emulsion due to the concentration of the salt (Rodeo). After 24 hours standing, it separated into two layers.
However the two layers mixed completely upon two to four inversions and produced a stable emulsion that could be used within a few hours.

A 4.0 g sample of the above emulsion (obtained after storage for 72 hours and inverting 2-4 times just before withdrawing the samples) was diluted to 101.9 g with deionized water which produced a clear solution.

The compositions were evaluated for rainfastness as described in Example 9. The results are set forth in Table 5. As shown, Latron B1956 exhibited an enhanced wash-off effect for a.i.-1 rather than improving its retention. The inventive composition exhibited an 80% higher retention of the a.i.-1 under the conditions of the experiments.

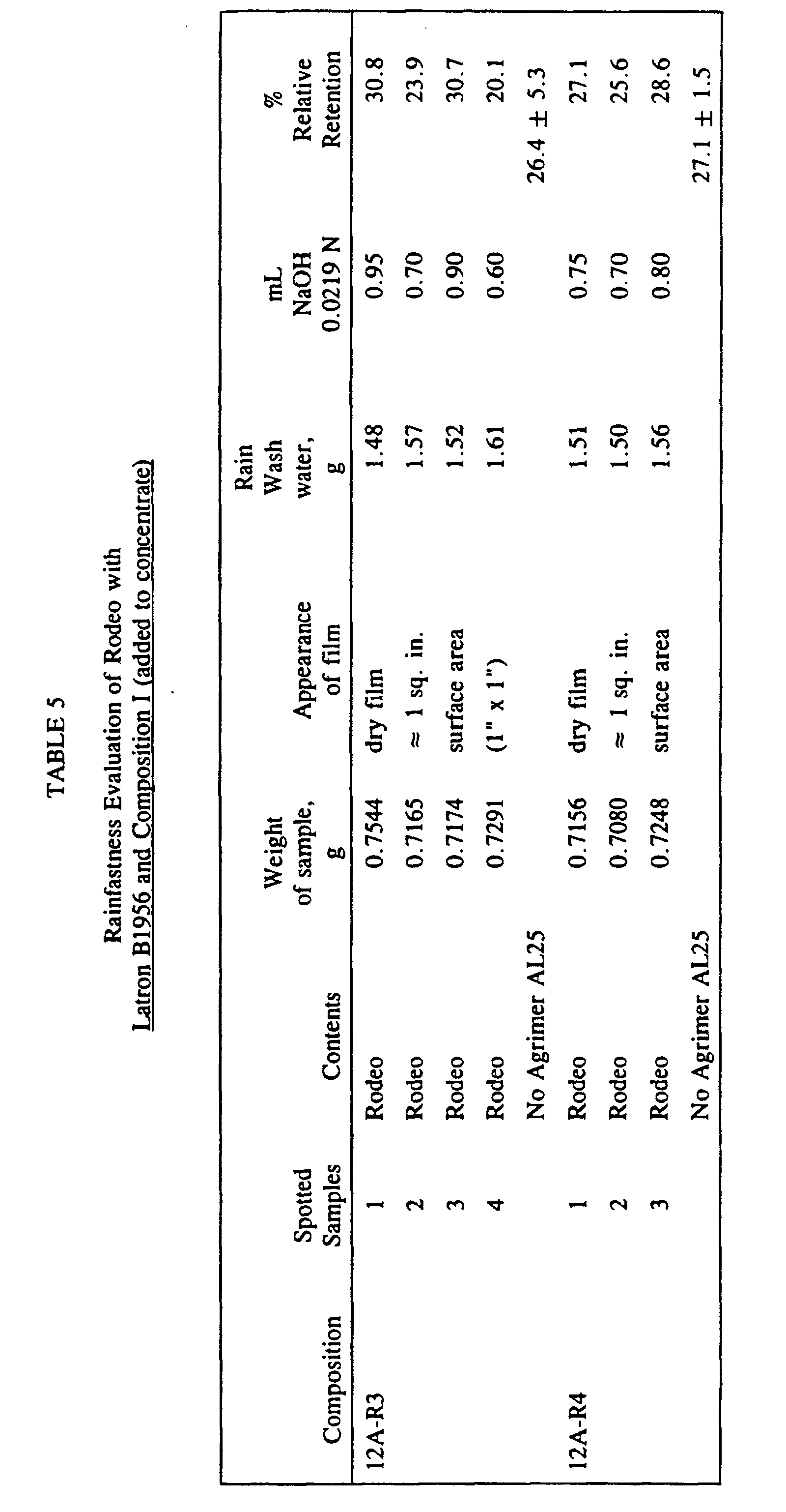

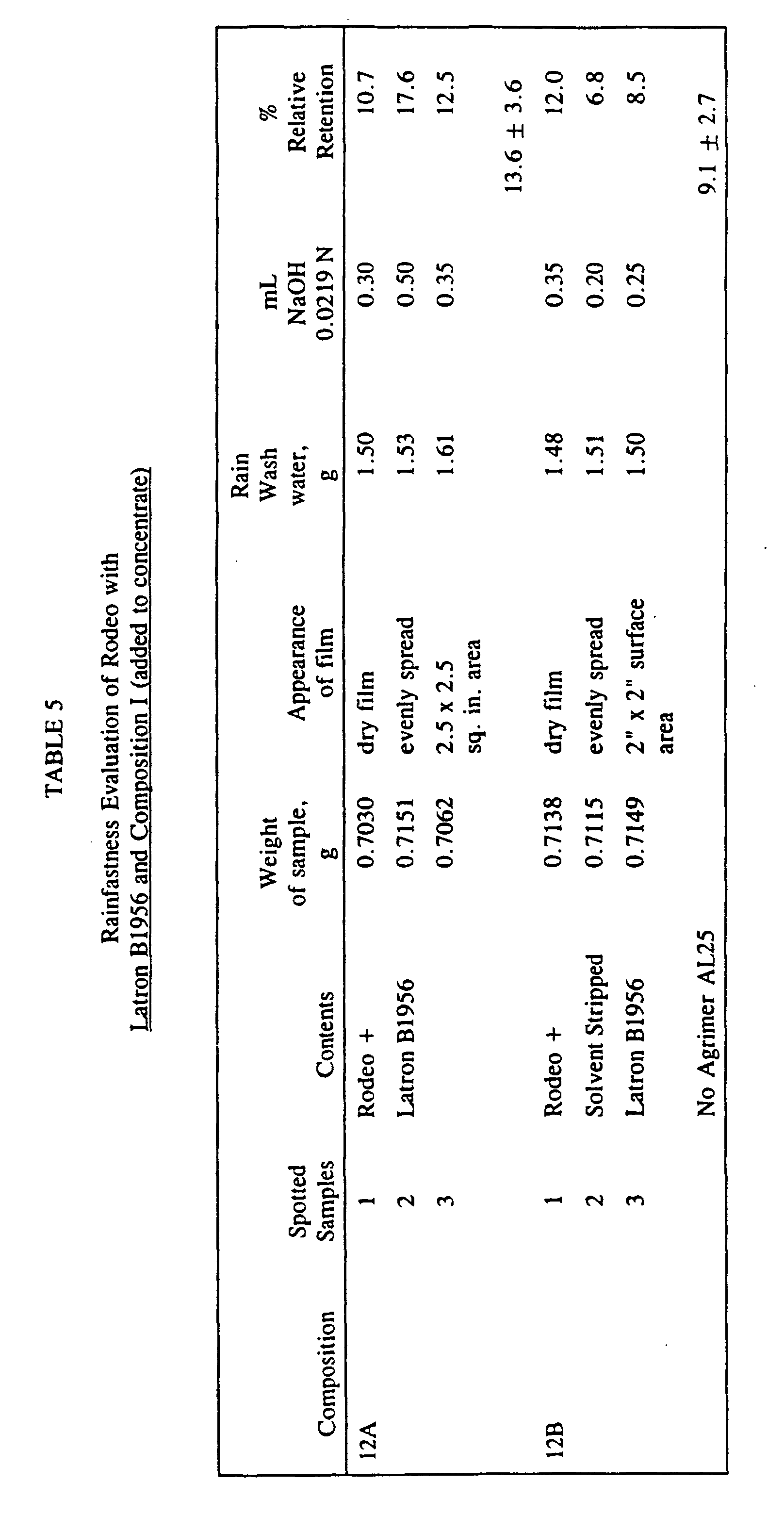

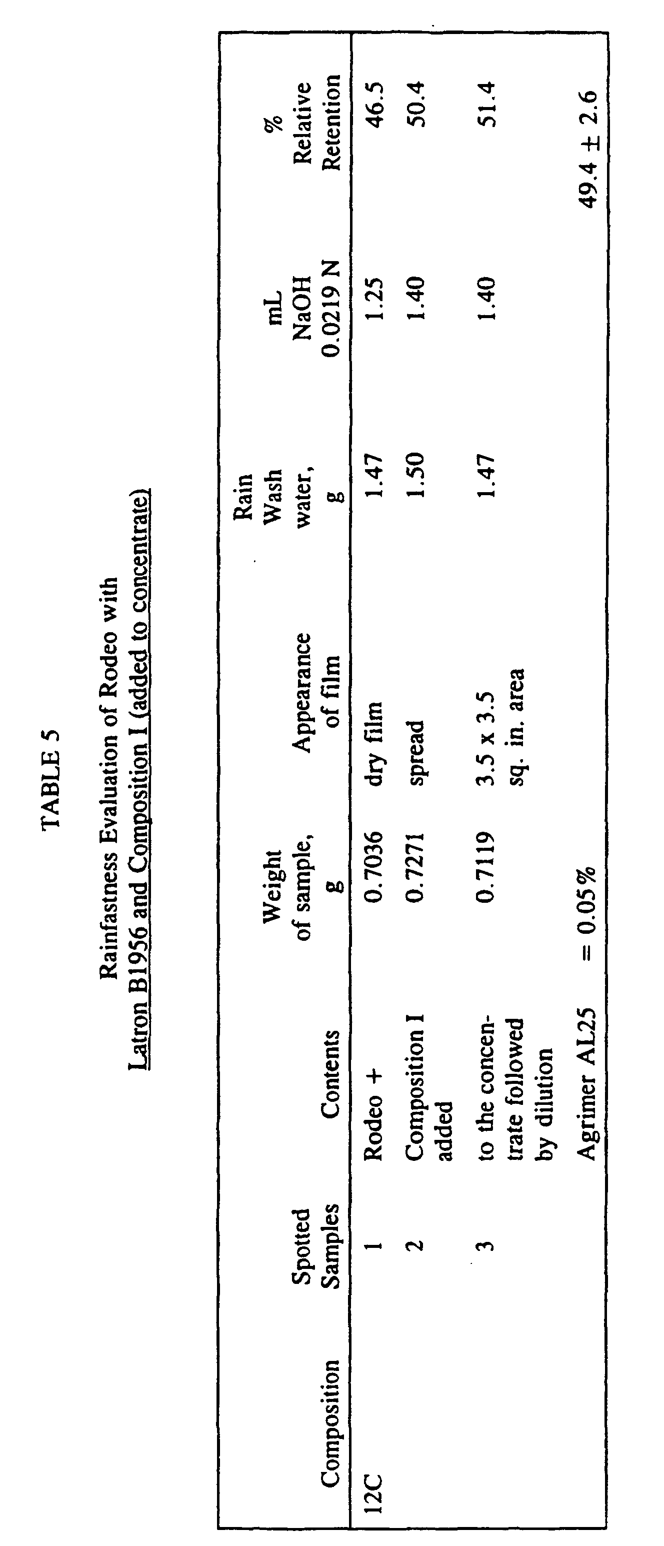

### Example 13

Rainfast evaluations for the inventive composition with pendimethalin (a.i.-2) were carried out. The a.i.-2 was in the commercial formulation Prowl. The procedure of Example 9 was followed. The a.i.-2 retention was determined by measuring the differential absorbance of the wash solution and EtOH at λ = 238.5 nm. The following compositions were prepared for comparative evaluation.
- 13A:: 1 g of Prowl was diluted to 100 ml with deionized water which produced a stable emulsion having an a.i.-2 concentration of 0.42%. An emulsion rather than a single phase was formed because pendimethalin is water insoluble. However, this does not affect the improvements obtained with the inventive composition.
- 13B:: 1 g of Prowl was mixed with 0.13 g of Composition I, Example 2, and diluted to 100 g to form an emulsion containing 0.42% pendimethalin and 0.02% Agrimer AL25 which was well dispersed in the medium.
- 13C:: Example 13B was repeated using 0.33 g of Composition I, to produce an emulsion containing 0.42 % pendimethalin and 0.05% Agrimer AL25.
- 13D:: Example 13B was repeated using 0.66 g of Composition I, producing an emulsion containing 0.42% a. i.-2 and 0.1% Agrimer AL25.

### Details of the Procedure:

Emulsions were prepared as above from 13A, 13B, 13C, and 13D. While stirring, aliquots were taken. 1 g of each aliquot was diluted with EtOH to 100 g, and further diluted 10X to 50X with EtOH. UV spectra of the diluted samples were obtained. The absorbance readings at λ = 238.5 nm for the samples fell in the range from 0.77 to 0.80, and the corresponding concentrations of the a.i.-2 were calculated to be 0.476%-0.492%. These values were used as the 100% retention values. The concentrations were calculated using a least squares line constructed using standard solutions of pendimethalin in EtOH. The least squares formula used was:$\text{absorbance = 0.080579 x concentration (ppm) + 0.0008785.}$

Glass plates were spotted in triplicate with about 0.3 g of the above emulsions. Films were formed by drying under ambient conditions for 72 hours. The films were rainwashed using water from an aspirated spray bottle. The remaining films were washed with 50-100 ml EtOH and the washings were collected. The absorbance of the ethanol solutions at λ = 238.5 nm were measured, and the % retention was calculated. The results are summarized in Table 6. The data shows that when Agrimer AL25 is used in the form of Composition I, much higher retention of the a.i.-2 is obtained. (64% Ex. 13C as compared to 21.3% for control Ex. 13A.)

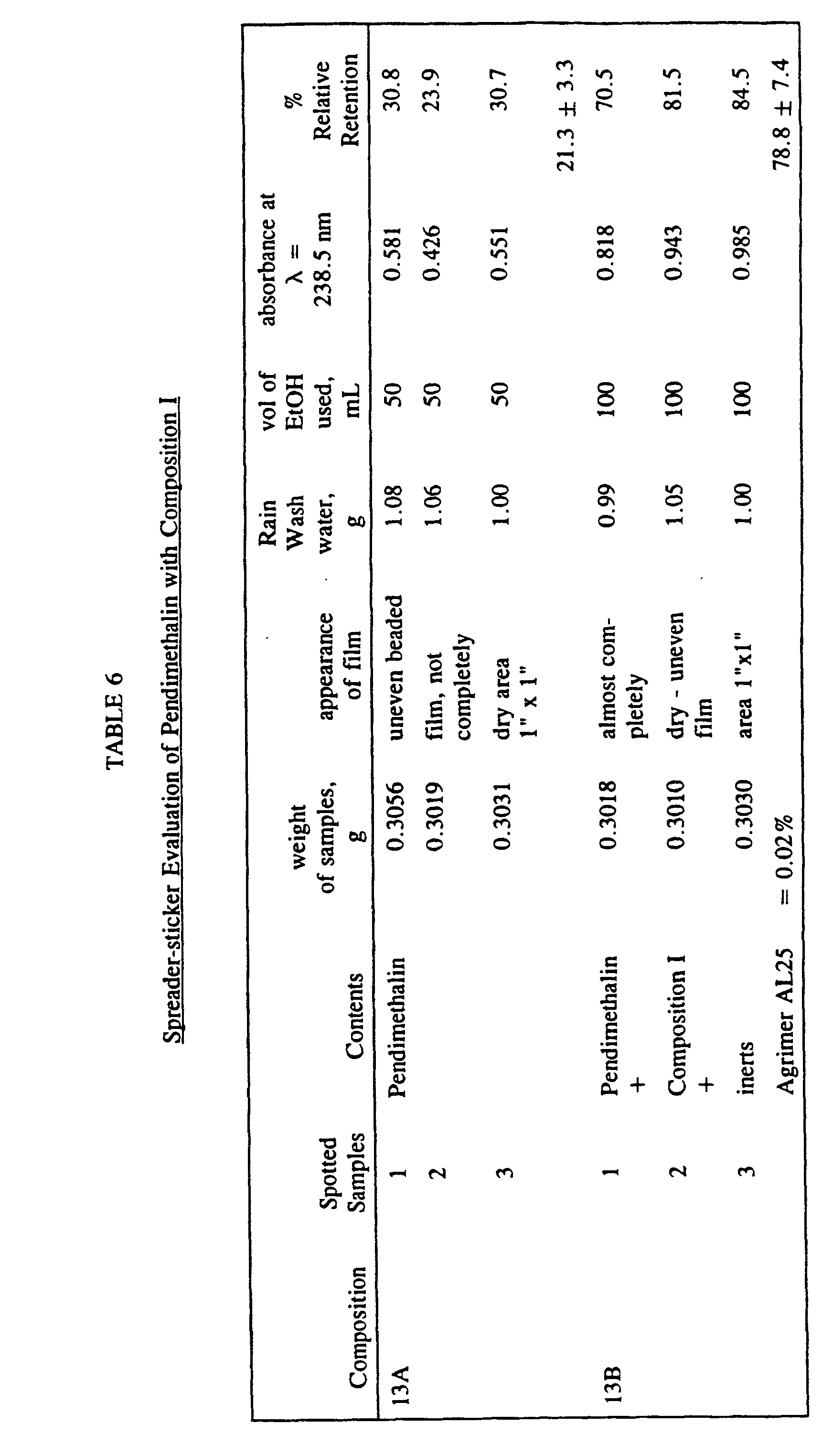

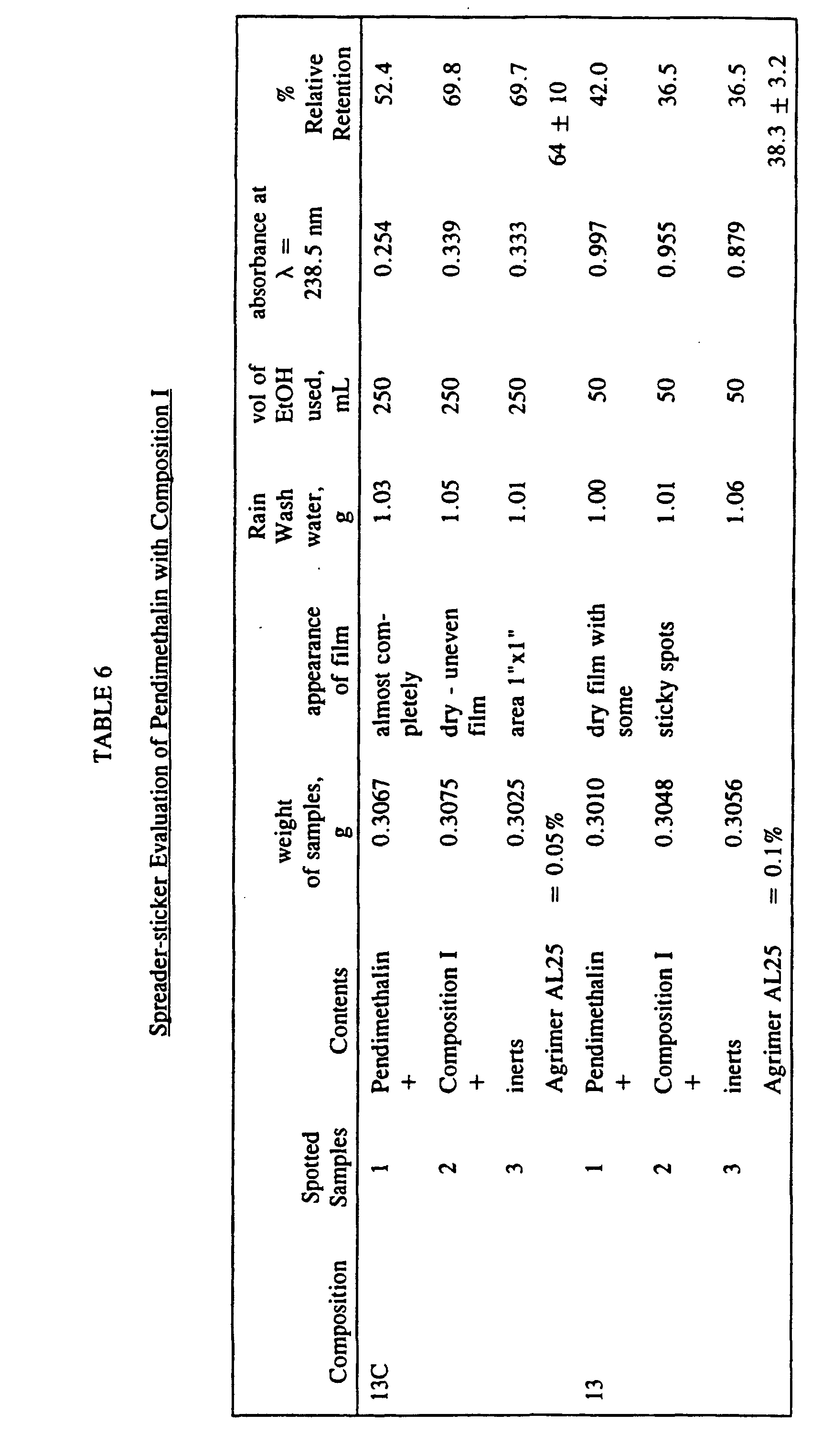

### Example 14

An evaluation of the rainfastness of compositions of Latron B 1956 and pendimethalin were carried out. Example 13 was repeated using Prowl and commercial Latron B1956. The following compositions were prepared:
- 14A:: 1 g of Prowl was diluted to 100 g with deionized water to produce a uniform emulsion.
- 14B:: 1 g of Prowl was mixed with 0.1 g Latron B1956 and diluted to 100 g with deionized. water to produce a uniform emulsion.
- 14C:: Example 14B was repeated using 0.2 g Latron B1956 in the place of 0.1 g Latron B1956.

In Example 13, all spots were dried outside the hood and the spray bottle was held about 2.5 to 5 cm (1 to 2 inches) away from the spot where 1 g of rain wash water was used.

In Example 14, all spots were dried inside the hood and the spray bottle was held about 10 cm (4 inches) away from the spot where 2 g of rainwater was used.

The results of the evaluation of Latron are summarized in Table 7.

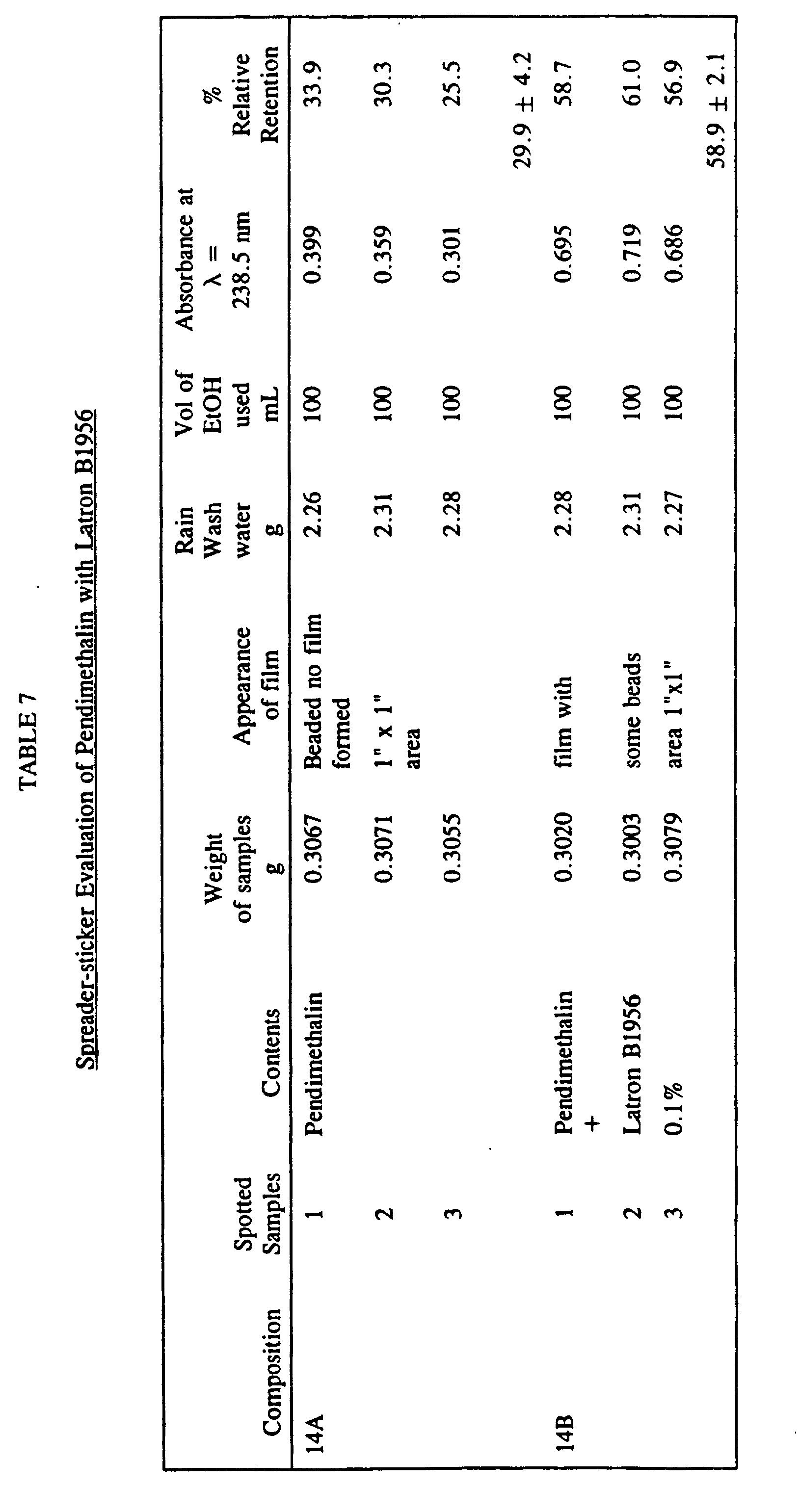

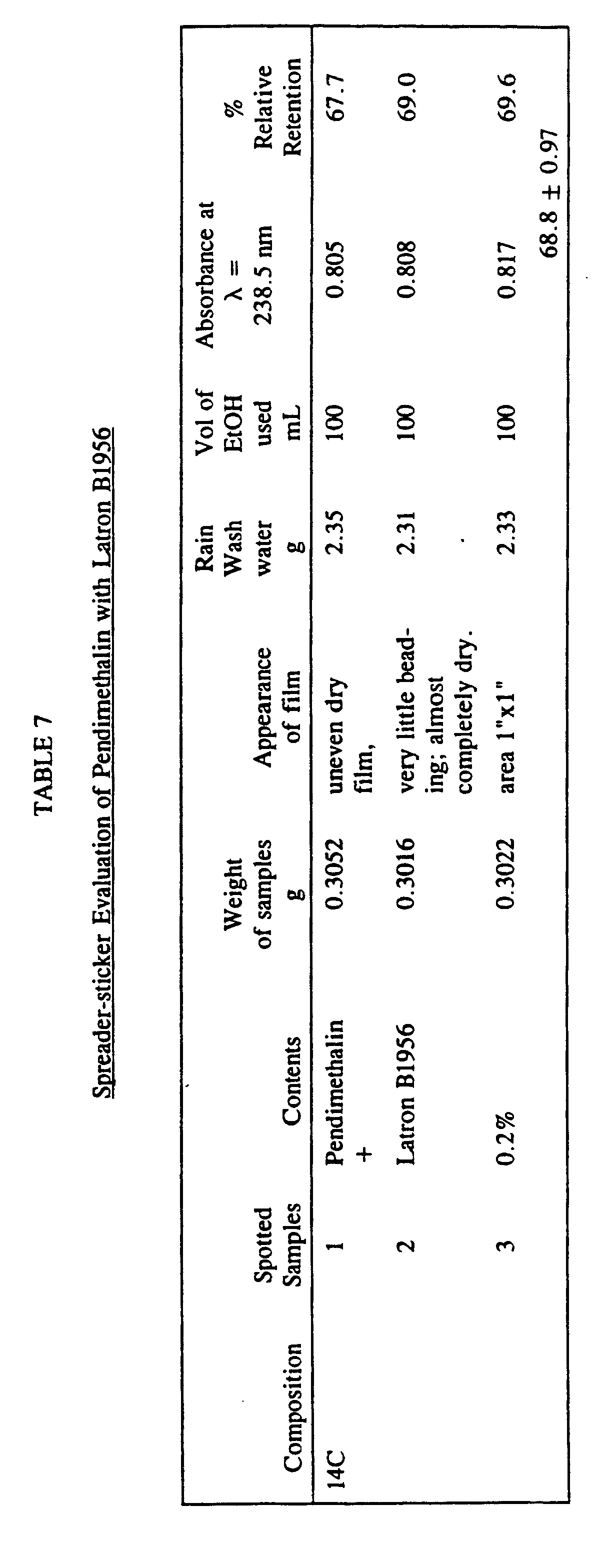

### Example 15

### Preparation of Modified Matrix

The following weighed ingredients were introduced into a 113 g (4 ounce) stoppered glass bottle:

| | |
|---|---|
| N-methylpyrrolidone | 20.0 g |
| N-octylpyrrolidone | 20.01 g |
| N-dodecylpyrrolidone | 13.01 g |
| Aqueous solution (29%) | 45.9 g |
| Sodium dodecylsulfate | |
| TOTAL | 98.92 g |

The following composition was similarly prepared except that N-methylpyrrolidone was not included. '

The following compositions were obtained:

| Compositions: | 15A | 15B |
|---|---|---|
| N-methylpyrrolidone | 20.22% | 0 |
| N-octylpyrrolidone | 20.22% | 40.30 |
| N-dodecylpyrrolidone | 13.15% | 13.40 |
| Sodium dodecylsulfate | 13.45% | 13.40 |
| Water | 32.96% | 32.90 |
| TOTAL | $\overline{\text{100.0%}}$ | $\overline{\text{100.0}}$ |

Composition 15A was a single-phase clear solution, while composition 15B separated into two layers on standing. Composition 15A was diluted 1/10, 1/50, 1/100 with deionized water. All of these diluted three samples were clear when observed up to 24 hours.

Composition 15A was used as an alternate matrix to solubilize Agrimer AL25.

### Example 16

To 47.51 g of composition 15A, 2.5 g of solid Agrimer AL25 were added and stirred in an orbital shaker for 4 hours at 350 RPM. 50.03 g of a clear homogeneous solution were obtained. This solution was diluted with deionized water in ratios of 1/10, 1/500, and 1/1000. No separation was observed in the diluted solution after for 24 hours.

### Example 17

Example 16 was repeated but using 45 g of composition 15A and 5.00 g of Agrimer AL25. The resulting composition remained a homogeneous single phase solution. On dilution with deionized water at ratios of 1/10, 1/500, and 1/1000, no separation was observed.

### Example 18

Example 16 was repeated using 42.5 g of composition 15A and 7.51 g of Agrimer AL25. The resulting polymer solution was clear and homogeneous and did not show any separation on dilution with deionized water at ratios of 1/10, 1/500, and 1/1000.

### Example 19

A series of examples were carried out using various alkyl pyrrolidones, including N-methyl pyrrolidone, N-octyl pyrrolidone, and N-dodecyl pyrrolidone. The N-dodecyl pyrrolidone exhibits stronger surfactant properties whereas the N-octyl exhibits stronger wetting properties, i.e., it wets hydrophobic surfaces as evidenced by the Draves Wetting test. The N-methyl pyrrolidone, on the other hand, is a better solvent and also provides enhanced plant and leaf penetration properties. I have found that a particularly desirable composition contains about 0.1 to 10, and preferably, about 0.5 to 2 parts of N-dodecyl pyrrolidone, and 0.1 to 10, and preferably, about 0.5 to 2 parts of N-methylpyrrolidone to 1 parts of N-octyl pyrrolidone, respectively. This composition provides not only enhanced leaf penetration, but also, better spreading due to the N-dodecyl component. Additionally, this composition allows increased amounts of the polymer component to be included in a single phase system.

For this purpose, Example 16 was repeated using 40.03 g of composition 15A and 10.01 g of Agrimer AL25. A homogeneous polymer solution was obtained after stirring for 4 hours in an automatic orbital shaker. No separation on dilution with deionized water at ratios of 1/10, 1/500, and 1/1000 was observed.

Compositions of Examples 16, 17, 18, and 19 and results of dilution are shown in Table 8.

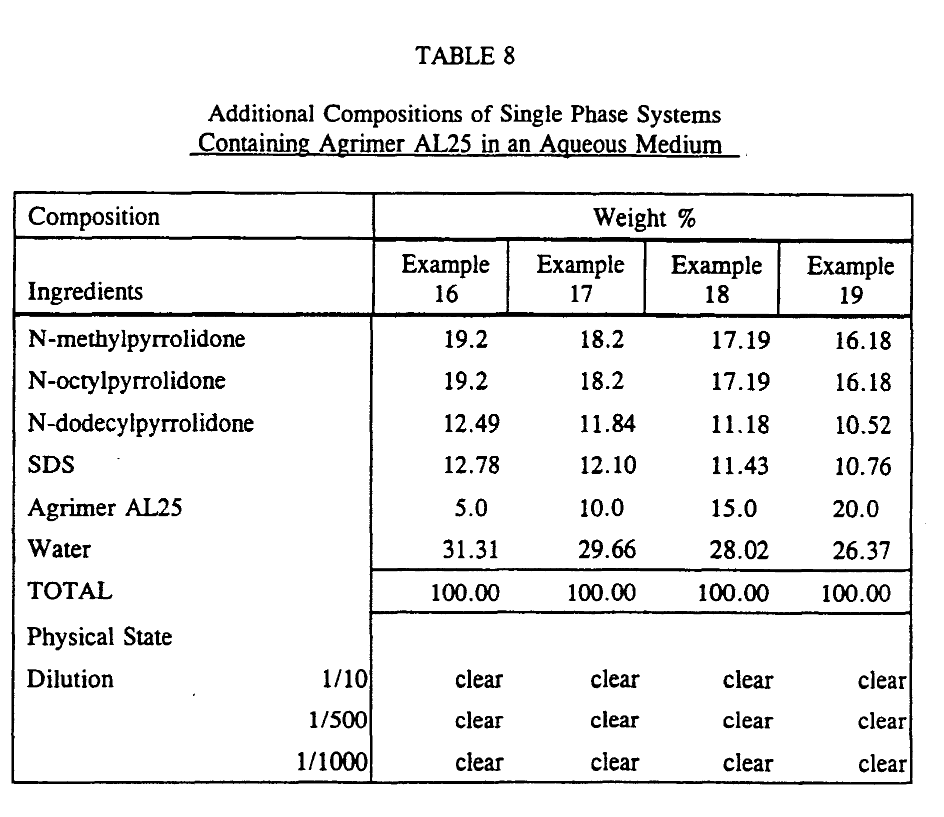

### Example 20

A series of microemulsions were prepared using the compositions of Examples 9B, 9D, 11B, 12C and 13B, with the exception that in place of Composition I, the composition of Example 19 was utilized. The compositions thus obtained were tested for rainfastness and produced results essentially similar to those depicted for the compositions 9B, 9D, 11B, 12C and 13B.

### Example 21

Compositions similar to those of Example 19 were prepared as follows:

The commercially available 50% solution of Agrimer AL25 in isopropylalcohol was mixed with the appropriate quantity of N-octyl pyrrolidone or N-dodecyl pyrrolidone. The mixture was then subjected to distillation conditions to distill off the isopropylalcohol either at atmospheric or under reduced pressure (17 mm Hg). When all of the isopropylalcohol was removed, the remaining ingredients constituting the compositions as shown were added. These compositions were evaluated for rainfastness in the manner described above by repeating Examples 9A, 9D, 13A and 13B. The results obtained were essentially similar to those of these examples. Accordingly, it is possible to add the polymer in an *in situ* manner as well as the dried polymer as described in connection with the preparation of the matrix.

### Example 22

A freeze dried formulation in accordance with the invention was prepared as follows:

Phosphonomethylglycine, 169 g (1 mole), was blended with NaHCO₃, 168 g (2 moles), both of these being solids, to produce a mixture which forms the sodium salt of the phosphonomethylglycine on contact with water. The mixture was ground to a fine powder.

80 g of this mixture was then blended with 20 g of the inventive composition (Composition I), followed by the addition of 50 g of water. This composition was subjected to freeze-drying and produced 70 g of a free-flowing powder product.

Upon mixing with water on the basis of 1 % by weight of the free-flowing powder product, a clear liquid (single phase) was obtained. This procedure thus allows for preparation of an inventive composition which is in dry form and thus easily transportable and which can, at the use or field site, be converted to a clear liquid upon the addition of water. It is particularly convenient as the dry inventive composition can be packaged in predetermined weights in a water-soluble container, e.g., a bag, which can simply be added to a dilution tank in the field.

If the active ingredient is water-soluble, then the use of bicarbonate or other soluble salt-forming reactant is not necessary. If effervescence is desired to enhance the rate of dissolution of the solid on the addition of water, an effervescing component, e.g., citric acid, or additional of bicarbonate may be added.

### Example 23

A series of tests were carried out to evaluate the effect of the inventive compositions on the efficacy of two commercially available herbicides, namely, Roundup and Gramoxone for Bahia grass and broad-leaf weed control.

The tests were conducted in Lake Alfred, Florida in citrus groves having trees of two years of age. The chemicals applied, conditions and application parameters at the time of application were as follows:

The concentrates were diluted so as to provide 454 g (1 pound) Glyphosate at 187 litres per hectare (20 gallons per acre) and 283 g (0.625 pound) Paraquat, also at 187 litres per hectare.

| | |
|---|---|
| Weather | clear |
| Humidity | 50% |
| Wind velocity | calm |
| Water source | well |
| pH | 7.1 |
| Volume of spray | 187 litres per hectare (20 gallons per acre) |
| Spray pressure | 241 kPa (= 35 psi) |
| Source of pressure | compressed air |
| Nozzle type/size | Teejet tips 8001 |
| Nozzle spacing | 25.4 cm (10 inches) |
| Type of sprayer | Tractor mounted boom sprayer |
| Tractor speed | 4.39 km/h (2.73 miles per hour) |
| Plot size | 13.7 by 3.0 meters (45 by 10 feet) |
| Number of treatments | 31 |
| Number of replications | 3 |
| Total number of plots | 93 |
| Stage of weed growth | 10 to 15 cm (4 to 6 inches) high |
| Major weeds | Broadleaf weeds: |
| | camphor weeks Florida pusley Jerusalem oak lambsquarters Pigweed Spanish needles tea weed |

The application was carried out in one day from 8:00 A.M. to 5:00 P. M.

Tables 9 and 10 show the effects of the treatment with controls and the inventive compositions on the growth of the respective weeds over a period of 63 days.

The herbicide was mixed with either inventive Composition I or Complex so as to give the active ingredient concentrations (a.i.-1) as indicated on Table 8. Complex had the following composition:

| | |
|---|---|
| Alkyl polyoxyethylene esters | |
| Polymerized resins and fatty acid | 16.6% |
| Reactive amines | 5.2% |
| Aromatic petroleum solvent | 4.9% |
| Inert solvents | 72.3% |

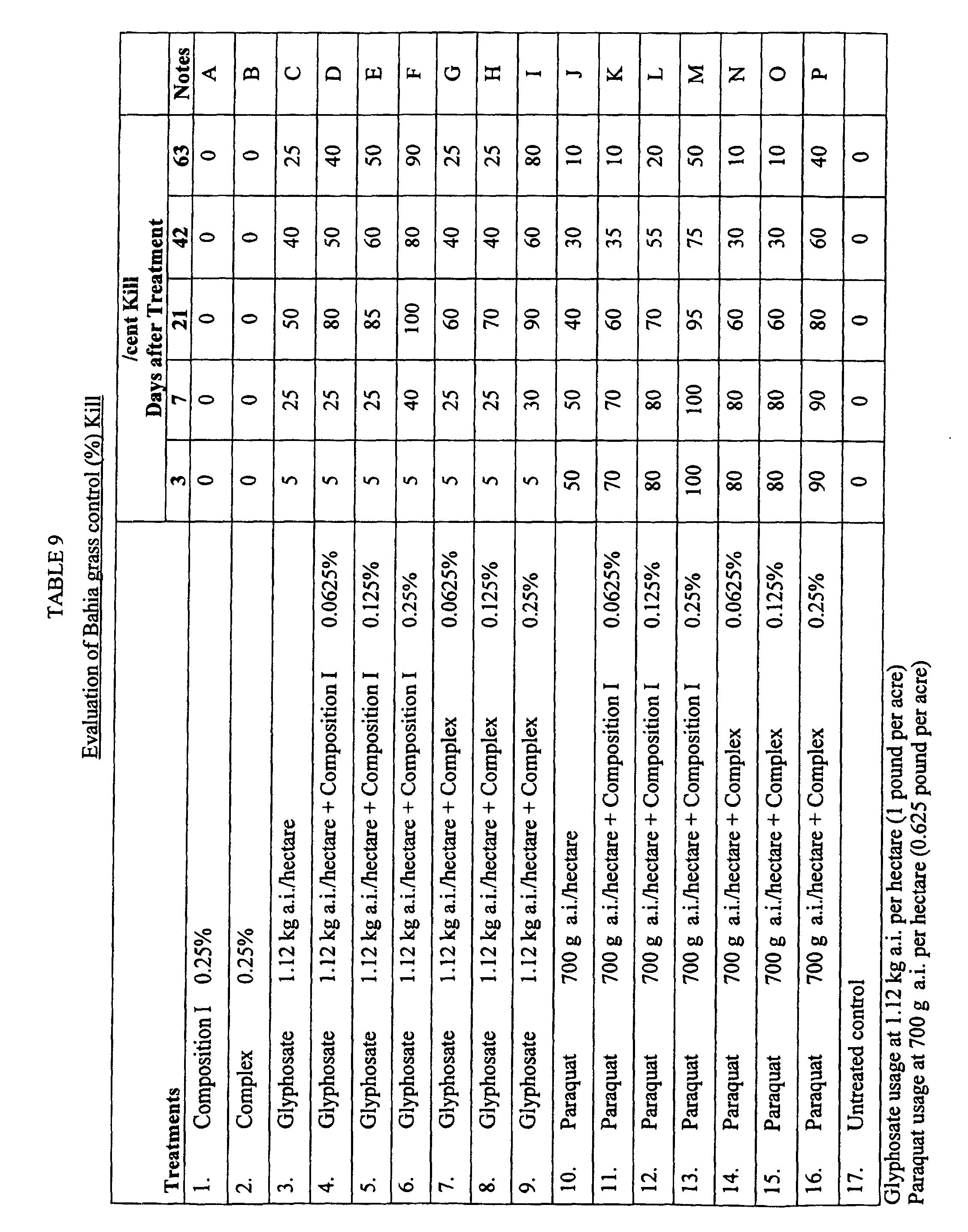

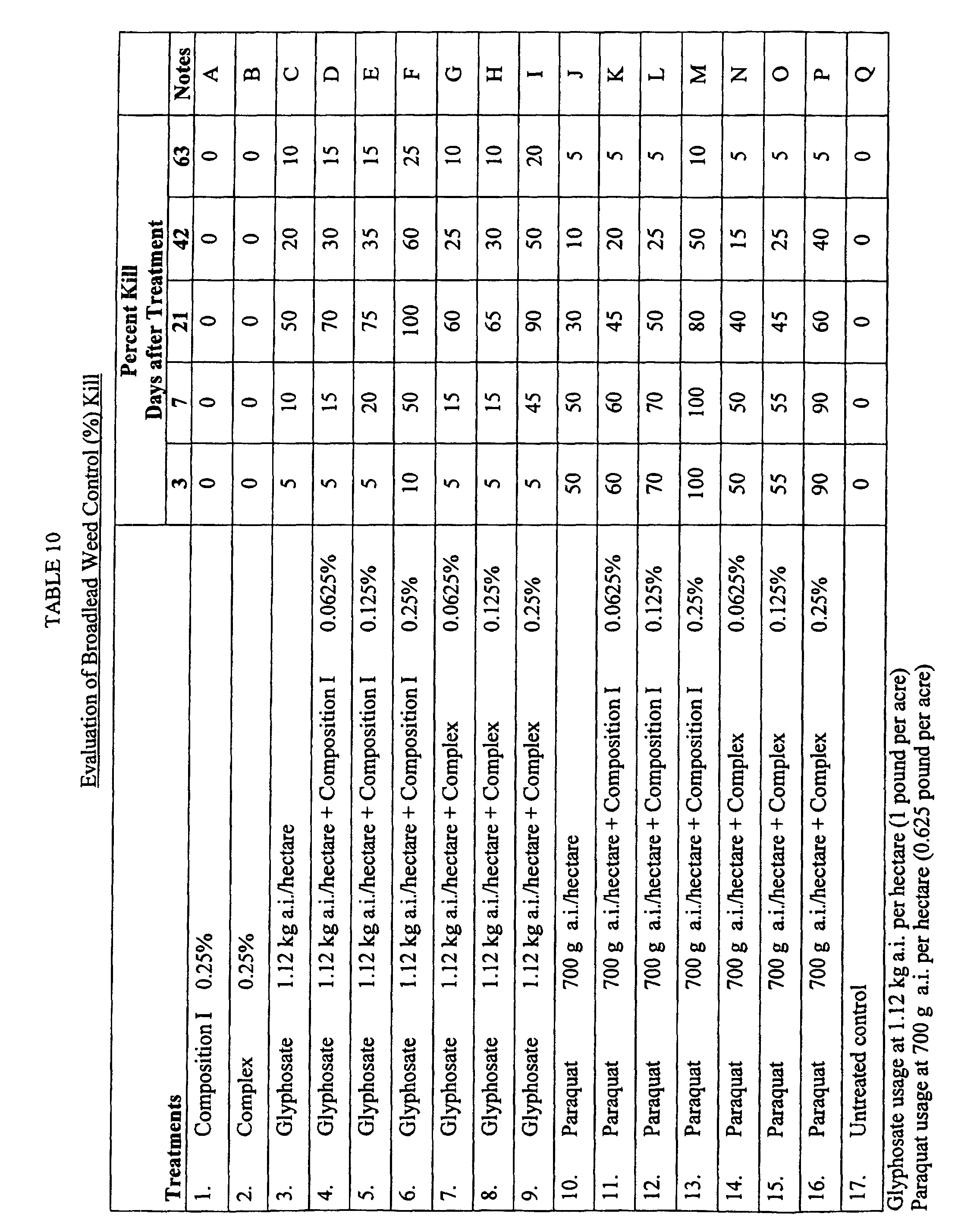

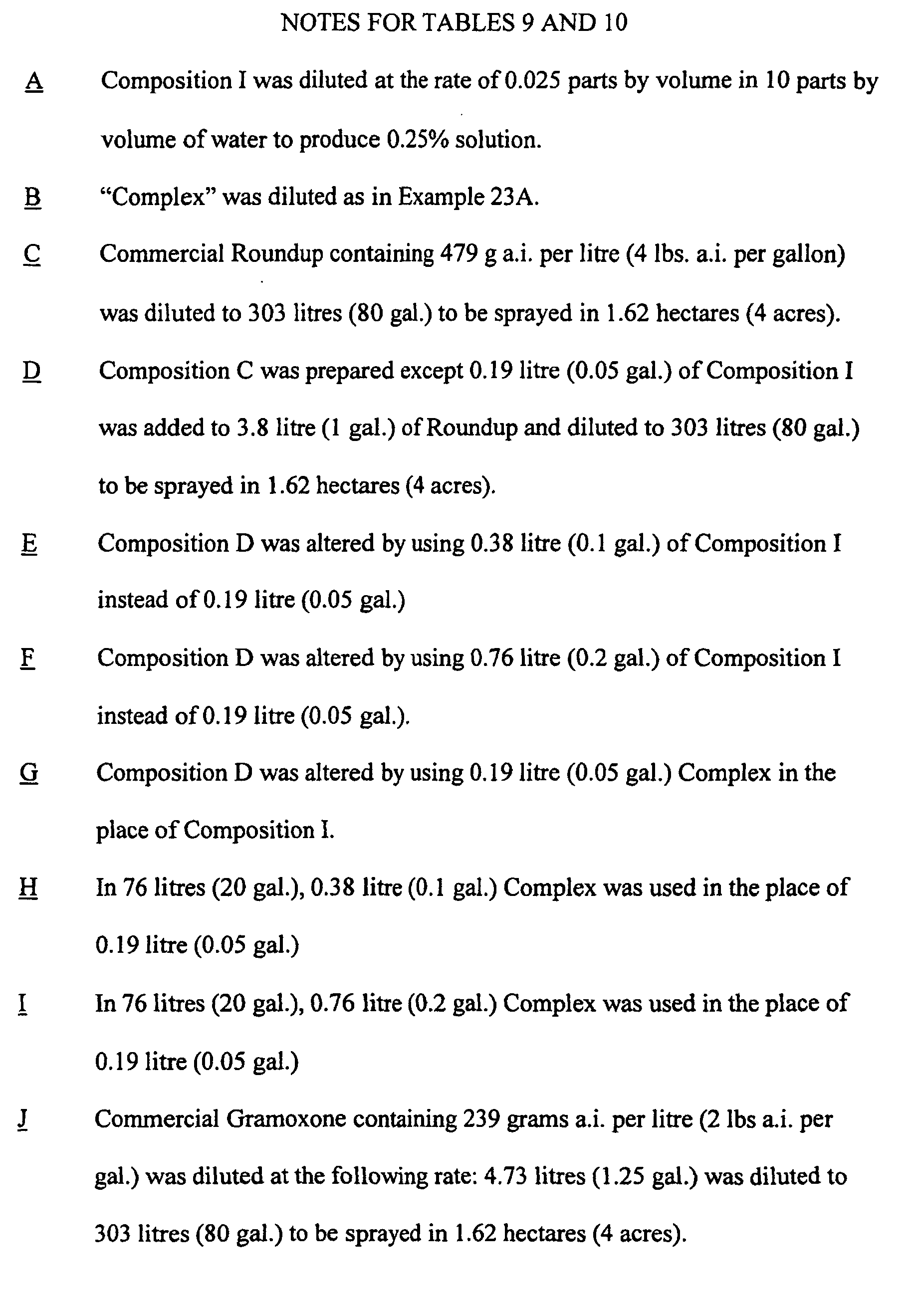

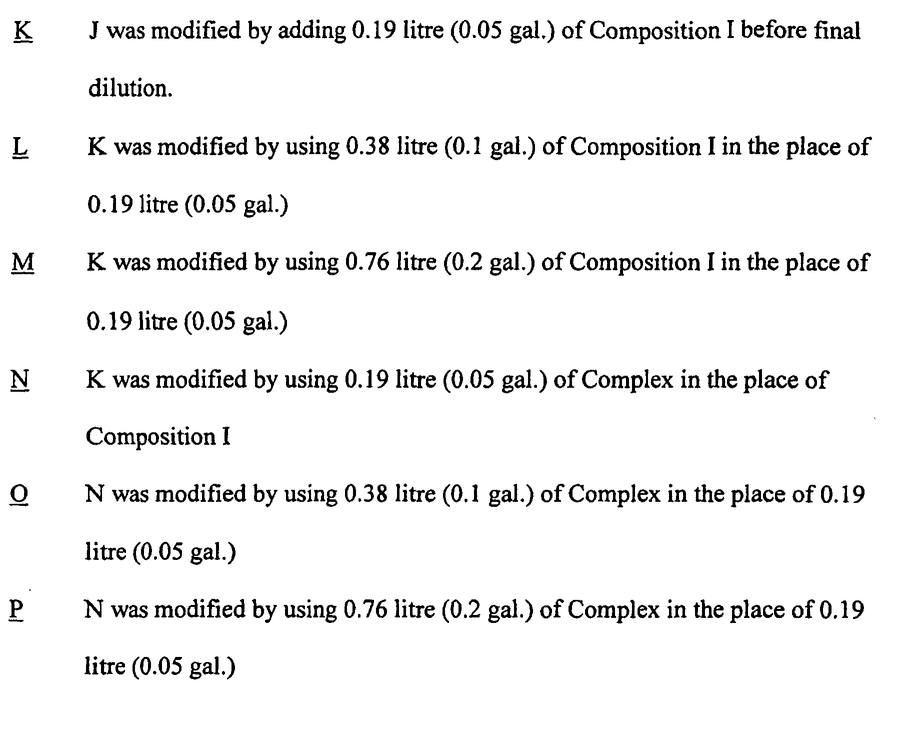

These results show that an enhanced biological efficacy is obtained when the a.i. is used as an adjuvant in the inventive composition. In particular the effect of the a.i. is accelerated and/or increased. Thus, with the present invention, it becomes possible to decrease the amount of active ingredient and still achieve a high efficacy.

### Example 24

Rainfastness evaluations were carried out for polymer films formed in accordance with the invention. These evaluations were carried out using compositions prepared from commercially available formulations. Such formulations conventionally are composed of an agriculturally active chemical (the active ingredient), surfactants and organic diluents as solvents. Consequently, with such commercial formulations, simply by adding the water insoluble polymer in accordance with the present invention, one can obtain the emulsion concentrate of the invention, the solvent and surfactant being present. It is noted that while the surfactant is already present in the commercial formulation as obtained and, consequently, cannot be removed, it is actually not necessary when used with the water-insoluble polymer according to the invention. However, its presence does not adversely affect the ability to produce a significant improvement in the rainfastness of the commercial formulation.

The general procedure used was as follows:
1. A commercial formulation of a given agriculturally active ingredient was admixed with a film-forming water-insoluble polymer.
2. The liquid mixture was then diluted to end use concentrations.
3. An appropriate dose (0.1 g to 0.5 g) was uniformly applied to a 15 cm by 15 cm (6 inch by 6 inch) glass plate uniformly as a 2.5 to 7.5 cm (1 to 3 inch) square patch. The patch was dried in a hood under ambient conditions for 48 to 72 hours. (3 samples were tested for each formulation.)
4. After a dry film was formed, a fine spray of water was applied to simulate 1.25 to 5 cm (0.5 to 2 inches) of rain wash off. The washings were collected in a waste jar.
5. The remaining washed patch was extracted with a solvent (ethanol is preferred) quantitatively into a 100 ml volumetric flask. If desired, appropriate dilutions of the ethanol extract were made.
6. The ethanol extract was subjected to ultraviolet spectra examination and the absorbance at a λ max. was determined (this value is designated X₁).
7. Blank samples of compositions III_{A}, III_{B}, III_{C}, III_{D}, III_{E}, III_{F}, and III_{G} were obtained by diluting each in ethanol to yield a solution containing 8 ppm of the active ingredient. The UV absorption of each was measured. These values were utilized to prepare a calibration chart. This value is designated X₂.
8. The percent retained was then determined by dividing X₁ by X₂ and multiplying by 100.
9. Blanks of the commercial formulations without polymer were run under identical conditions. The percentage was calculated.
10. For samples wherein the pesticide used is Pendimethalin, λ max. is 239 nm. 0.3 g of diluted samples were used for patches (6.45 to 12.9 cm² i.e. 1 to 2 square inches). The amount of wash-off water used was 1.5 to 2.5 g.
In all examples using an aromatic oil in the formulations, samples of the alcohol extract from the dried spot after the rain wash were completely evaporated in a vacuum oven to remove any residual aromatic solvent so as to avoid interference therefrom in the absorbance analysis.

Using the above general procedure, rainfastness evaluations for commercial pendimethalin formulations using Prowl as a commercially available emulsifiable concentrate of this active ingredient. It is an oil-based concentrate. This commercial formulation contains 42.0% of the active ingredient with the balance being surfactants and organic solvents. The compositions used were as follows:
- Composition III_{A} -: 1 g of Prowl was diluted with 100 ml of D.I. water to provide an end use composition containing 0.42% active ingredient.
- Composition III_{B} -: 90 parts of Prowl were mixed with solid Agrimer AL 25 (10 parts). 1 g of this mixture was diluted to 100 parts with D.I. water.
- Composition III_{C} -: The formulation of I_{A} (1 g diluted to 50 g with D.I. water).
- Composition III_{D} -: The formulation of I_{B} (1 g diluted to 50 g with D.I. water).
- Composition III_{E} -: This composition was the same as III_{B} except that Agrimer VA3 was used in place of Agrimer AL 25. Agrimer VA 3 is commercially available as a 50% solution in ethanol. In these examples, it was used in solid form obtained by stripping the ethanol from the solution under vacuum at low temperature - under 65°C). The resulting composition was obtained by diluting 1 g to 100 ml with D.I. water.
- Composition III_{F} -: 90 parts of Prowl were mixed with 10 parts of solvent-stripped Latron B1956. 1 g of this composition was diluted to 100 g with D.I. water.
- Composition III_{G} -: The composition was the same as composition III_{B} except that Agrimer AL 25 was replaced with Agrimer AL 30.

The results of the rainfastness formulations are shown in Table II.

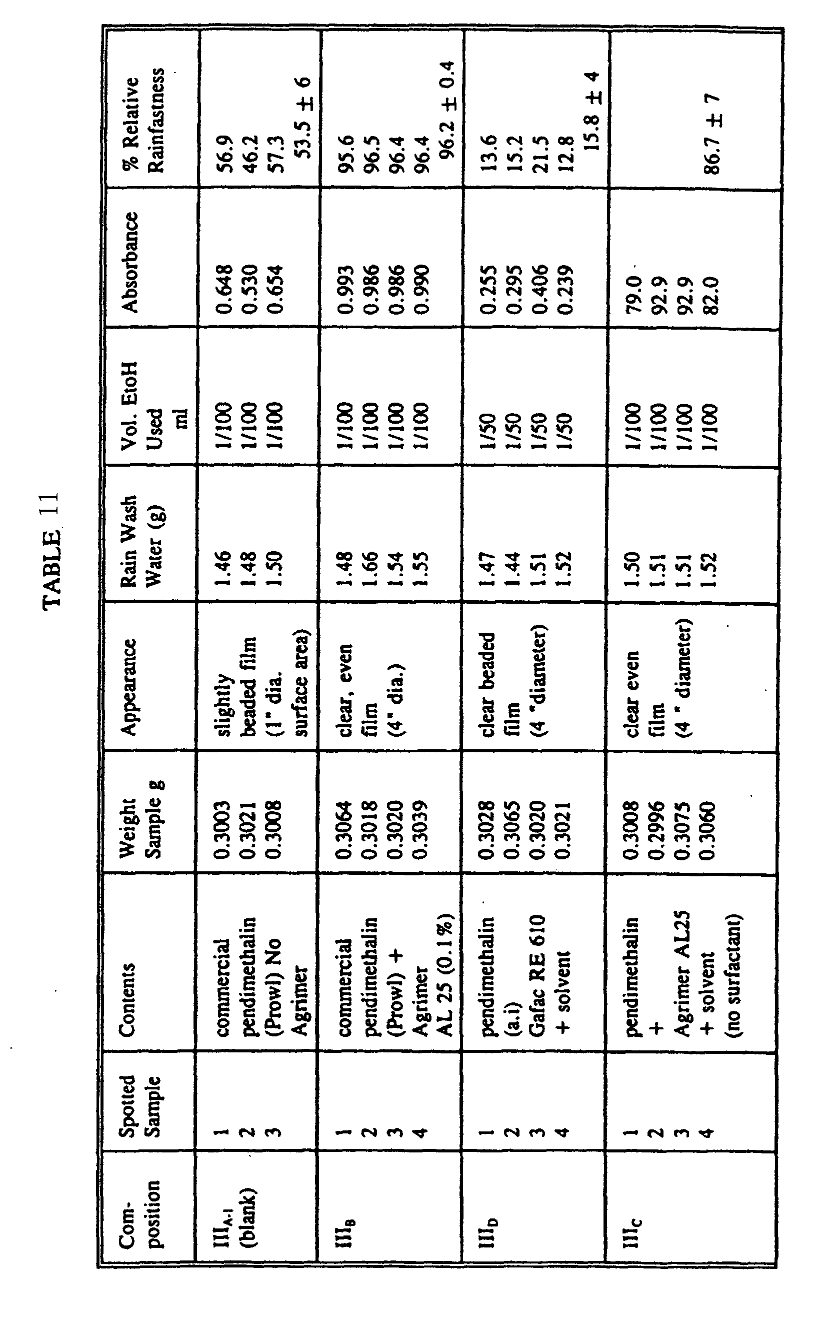

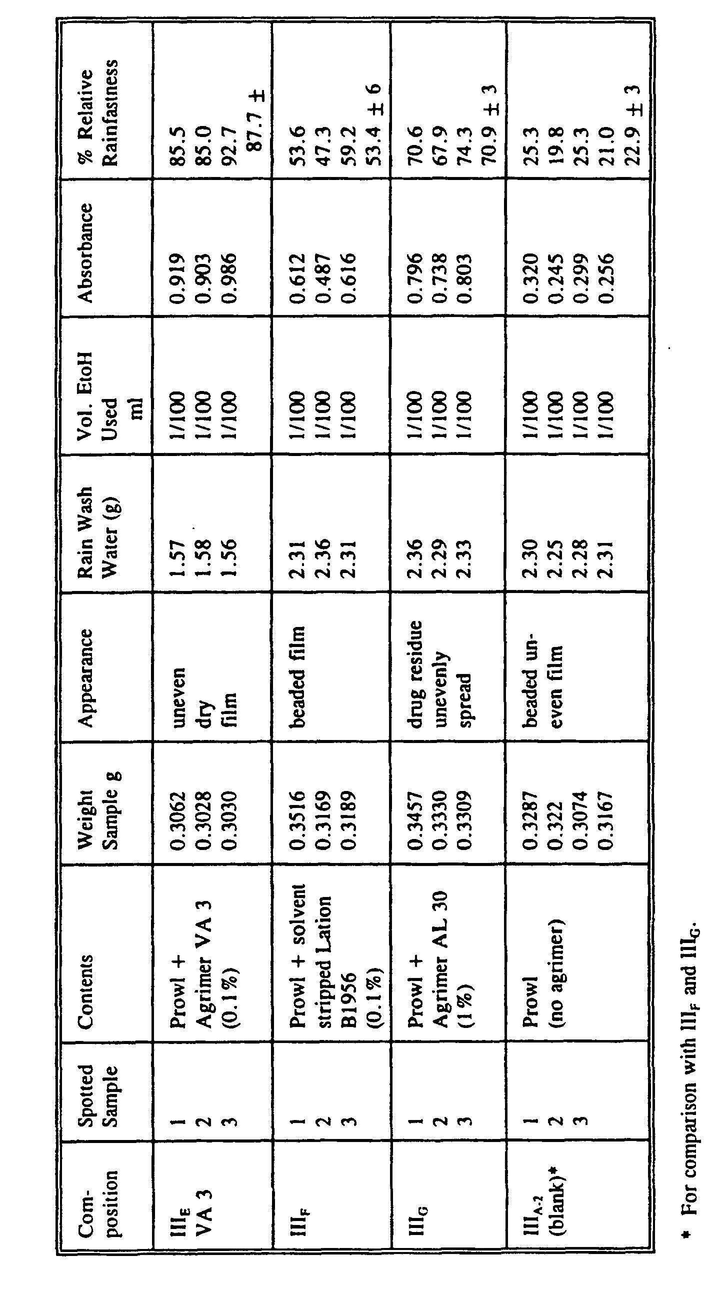

To test the validity of the procedure, compositions III_{A}, III_{B}, III_{C}, III_{D}, III_{E}, III_{F}, and III_{G} were diluted in ethanol to yield a solution containing 8 ppm of the active ingredient. The UV absorbance was measured at λ max. and all values (concentration of active ingredient) were within ± 2 % of the theoretical values.

### Example 25

The following compositions were prepared:
- Composition IV_{A} -: identical to composition III_{A}
- Composition IV_{B} -: same as composition III_{B} except that 2 parts of Agrimer AL 25 were mixed with 98 parts of Prowl and 1 g of this mixture was diluted to 100 g with D.I. water.
- Composition IV_{C} -: This was the same as composition III_{B} except 5 parts of Agrimer AL 25 were mixed with 95 parts of Prowl.

The rainfastness was evaluated using the experimental procedure of Example 24. The percent of active ingredient recovered from the formulations is shown in Table 12.

**TABLE 12**

| Compositions | % Agrimer AL 25 in the final dilution | % a.i. recovered |
|---|---|---|
| IV_{A} | 0 | 54.7 ± 6 |
| IV_{B} | 0.02 | 77.2 ± 2 |
| IV_{C} | 0.05 | 80.1 ± 7 |

### EXAMPLE 26

The following formulations were prepared:
- Composition V_{A} -: 14.61 g of dried Agrimer AL 25 were dissolved in 64.29 g of Exxon Aromatic 150, 14.61 g N-octyl pyrrolidone were added, followed by the addition of 6.49 g of a surfactant, Alcasurf CA. The mixture was stirred for 40 minutes in an orbital shaker to produce a clear solution. The composition can also be prepared by using 29.2 g of a 50% isopropyl alcohol solution of the polymer (Agrimer AL 25) and 14.61 g of N-octylpyrrolidone with 64.3 g of Exxon Aromatic 150 (or other higher boiling diluents). The isopropyl alcohol may be separated by evaporation under atmospheric or reduced pressure. 6.49 g Alcasurf CA, is then added to produce 100 g of the inventive composition.
- Composition V_{B} -: 14.6 g of Agrimer AL 30 were dissolved in 14.6 g of N-octyl pyrrolidone, 64.3 g of Exxon aromatic 150 and 6.5 g of Alcasurf CA. The mixture was stirred for 30 minutes to produce a clear solution.
- Composition V_{C} -: 15 g of Agrimer AL 25 (solvent-stripped) were dissolved in 85 g of Exxon aromatic 150.

- Composition V_{D} -: This composition was the same as composition V_{C} except that the Agrimer AL 25 was replaced by an equivalent amount of Agrimer AL 30 (solvent-stripped).

(The above-noted compositions are depicted in Table 13)

**TABLE 13**

| % WEIGHT COMPOSITIONS OF SOLVENT BASED POLYMER COMPOSITIONS: | | | | |
|---|---|---|---|---|
| Ingredients | V_{A} | V_{B} | V_{C} | V_{D} |
| N-octylpyrrolidone | 14.6 | 14.6 | - | - |
| Exxon Aromatic 150 | 64.3 | 64.3 | 85 | 85 |
| Ethanol | - | - | - | - |
| Agrimer AL25 | 14.6 | - | 15 | - |
| Agrimer AL 30 | - | 14.6 | - | 15 |
| Alcasurf CA | 6.5 | 6.5 | - | - |
| Total | 100.0 | 100.0 | 100 | 100 |

Compositions V_{A} and V_{B} were diluted with D.I. water or 342 ppm hard water to produce stable emulsions at dilution rates of 1/10, 1/20, 1/50, 1/100 and 1/1000. The emulsions produced from composition V_{A} were more stable than those from composition V_{B}. Hard water produced more stable emulsions than D.I. water. Formulation of stable emulsions in water makes it possible to use the above compositions in both oil based and water based active ingredient formulations. It is noted that compositions V_{A} and V_{B} are particularly advantageous for use in oil base active ingredient concentrates in aqueous medium.

### EXAMPLE 27

Rainfast evaluations using the procedure of Example 24 hereinabove were carried out for compositions V_{A}, V_{B}, V_{C}, and V_{D} as well as at Latron B 1956 for comparison purposes. The compositions used were as follows:
- Composition VI blank: This was essentially the same as composition III_{A}.
- Composition VI_{A} -: 1 g of Prowl was mixed with 0.67 g of composition V_{A} and then diluted to 100 g with D.I. water.
- Composition VI_{B} -: 1 g of Prowl was mixed with 0.67 g of composition V_{B} and then diluted to 100 g with D.I. water.
- Composition VI_{C} -: 1 g of Prowl was mixed with 0.67 g of composition V_{C} and then diluted to 100 g with D.I. water.
- Composition VI_{D} -: 1 g of Prowl was mixed with 0.67 g of composition V_{D} and then diluted to 100 g with D.I. water.

Formulations VI blank, VI_{A}, VI_{B}, VI_{C} and VI_{D} were evaluated for rainfastness of the pendimethalin using 0.32 - 0.34 g for spotting and 2.2 to 2.4 g of wash water to simulate rain. The evaluation showed that formulations VI_{A}, VI_{B}, VI_{C} and VI_{D} exhibited increased retention of the active ingredient as compared to the blank. The effectiveness was in the following decreasing order:${\text{VI}}_{\text{A}} {\text{(38.7 ± 6) > VI}}_{\text{B}} {\text{(30 ± 1) > VI}}_{\text{D}} {\text{(23 ± 4) > VI}}_{\text{C}} \text{(19.4 ± 3) > VI (12 ± 1)}$

The foregoing represent the averages of three runs for each composition.

In the above experiment, the pump spray equipment used, i.e., force of spray, to simulate rain was different from the procedure outlined in Example 24. This could explain the lower retention.

The evaluation of Latron B1956 as a tank mix additive showed that the inventive formulations produced 60 to 80% higher retention at the same level of application as the Latron B1956 containing composition.

### EXAMPLE 28

A series of formulations was prepared containing commercial carbaryl (Sevin) and evaluated for rainfastness. Sevin is a formulation containing 27% of carbaryl as an active ingredient. The compositions were as follows:
- Composition VII blank: 1 g of commercial Sevin was diluted to 200 g with D.I. water to produce an emulsion of about 0.14% active ingredient.
- Composition VII_{A} -: Same composition as VII blank except that 0.27 g of composition V_{A} was added prior to dilution.
- Composition VII_{B} -: Same composition as VII_{A} except that 0.67 g of composition V_{A} were added.
- Composition VII_{C} -: Same composition as VII_{A} except that composition V_{A} was replaced with composition V_{B}.
- Composition VII_{D} -: Same as composition VII_{B} except that composition V_{A} was replaced by composition V_{B}.

- Composition VII_{E} -: Same as composition VII_{B} except that composition V_{A} was replaced by composition V_{C}.
- Composition VII_{F}-: Same as composition VII_{B} except that composition V_{A} was replaced by composition V_{D}.

These formulations were evaluated using the same procedure as set for in Example 24. Specifically, 0.45 - 0.54 g of the sample were spotted onto glass plates and 1.5 - 1.7 g of water was used to simulate rain wash. The λ max. used for the evaluation was 279 nm and the least square line was used according to the following formula:$\text{absorbance = 0.03276 X concentration (ppm) - 0.016416.}$

The results of the evaluations are shown in Table 14.

**TABLE 14**

| SUMMARY RESULTS OF RAINFASTNESS FOR CARBAMYL ("SEVIN") FORMULATIONS | | |
|---|---|---|
| COMPOSITION | % AGRIMER | % A.I. RECOVERED |
| VII (blank) | 0 | 39.2 ± 4 |
| VII_{A} | Agrimer AL 25 (0.02%) | 55.4 ± 7 |
| VII_{B} | Agrimer AL 25 (0.05%) | 59 ± 8 |
| VII_{C} | Agrimer AL 30 (0.05%) | 63 ± 7 |
| VII_{D} | Agrimer AL 30 (0.05%) | 62 ± 9 |
| VII_{E} | Agrimer AL 25 (0.05%) | 56 ± 6 |
| VII_{F} | Agrimer AL 30 (0.05%) | 59.6 ± 8 |

### EXAMPLE 29

It is noted that formulation V_{A} is particularly useful as an adjuvant for oil based concentrates. Such oil based concentrates (concentrates based on a non-aqueous hydrophobic solvent) normally are composed of:
active ingredients - 10 to 60 percent;
surfactants (wetting agents and emulsifiers) - 1 to 30 percent;
hydrophobic non-aqueous solvents - 10 to 80 percent; and optionally,
defoamers, rheology modifiers and the like as needed 0 to 5 percent.

It exhibits enhanced efficacy with the active ingredient Fusilade EC when used at a 0.2 % concentration in the final dilution. Also, increased biological activity with chloropyrifos, an insecticide, was observed when composition V_{A} was used with an oil based concentrate for this active ingredient at a 0.2 % concentration in the final dilution.

### EXAMPLE 30

The compositions of the present invention, for example, Compositions V_{A} and V_{B} can be used as granulating fluids to incorporate the water-insoluble polymers in solid water-dispersible granular formulations. A typical formulation is as follows:

| Active ingredient (Atrazine) | 82.3 % by weight |
|---|---|
| Binder (polyvinyl pyrrolidone - optional) | 3.0 |
| Dispersant | 3.0 |
| Wetting agent | 1.5 |
| Defoamer | 0.2 |
| Composition V_{A} or V_{B} | 10.0 |

Typical examples of the dispersants, wetting agents, and defoamers are: Morwet D-425, alkylated naphthalene sulfonate sodium salt, Morwet EFW which is a proprietary mixture of sulfated alkylcarboxylate and a sulfonated alkylnaphthalene sodium salt, and FOAMASTER soap L which is a proprietary soda soap.

In such a composition, the aromatic oil diluent can be dispensed with. The water-dispersible granules are prepared by charging the weight ingredients (200 g) in a V-blender and mixing for 10 to 30 minutes. The charge is then loaded to a 24 inch pan granulator set at an angle of 50 to 55°. The pan is rotated at a speed of 15 rpm and granulation is accomplished by spraying the charge with water at an appropriate speed, typically, 20 to 30 ml of water is used over a period of 10 to 30 minutes. After granulation, the wet granules are dried at 40 to 50°C to reduce the moisture level to about 1 to 2 % . The dry granules can be sieved to segregate them into different size fractions as desired. Undersized granules can be reformulated by recycling to the process. The granules can also be prepared by using a conventional extrusion process with a suitable extruder.

## Claims

1. An emulsion concentrate comprising:
a) 2 to 90% by weight of a solvent selected from the group consisting of long-chain alkyl pyrrolidones, synthetic or naturally occurring hydrophobic oils, and mixtures thereof;
b) 1 to 60% by weight of a water insoluble graft polymer of N-vinyl pyrrolidone and an alpha-olefin wherein the N-vinyl pyrrolidone is present in at least 20% on a weight basis; and
c) 1 to 85% by weight of a surfactant.

2. The concentrate of claim 1 wherein the solvent is a long chain alkyl pyrrolidone having the formula 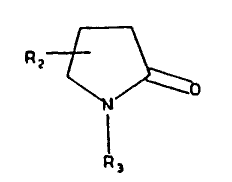 wherein R₂ is hydrogen or alkyl having from 6 to 14 carbon atoms and R₃ is alkyl having from 6 to 14 carbon atoms with the proviso that at least one of R₂ or R₃ must contain at least 6 carbon atoms and the sum of the carbon atoms in R₂ and R₃ cannot exceed 14, preferably R₂ is hydrogen and R₃ is C₈ or C₁₂, or a synthetic or naturally occurring oil having a hydrophobic character or having a fractional dispersive solubility parameter of greater than 70%.

3. The emulsion concentrate of claim 1 comprising a said solvent which is N-octyl pyrrolidone or a crude oil distillate having an average boiling point of at least 120°C, or mixtures thereof.

4. The concentrate of claim 1 wherein the water insoluble polymer is a graft copolymer of N-vinylpyrrolidone and an α-olefin containing 16 to 20 carbon atoms.

5. The concentrate of claim 1 wherein the water insoluble polymer is a graft copolymer of N-vinylpyrrolidone and a C₂₀ α-olefin in a 50:50 weight ratio.

6. The concentrate of claim 1 wherein the water insoluble polymer is a graft copolymer of N-vinylpyrrolidone and a C20 α-olefin in a 20:80 weight ratio.

7. The concentrate of claim 1 wherein the solvent is 10-20% by weight N-octyl pyrrolidone , or 50-70% by weight of the hydrophobic oil, or mixtures thereof, the water insoluble polymer is present in an amount from 10 to 20% by weight and the surfactant is present in an amount from 5 to 10% by weight.

8. A concentrate of any one of the preceding claims further including an agriculturally active chemical.

9. A composition for application to agricultural crops comprising an emulsion which is a composition of claim 8 diluted at least 2-fold with water.

10. A method for producing a water-dispersible granule of an agriculturally active ingredient charging an agriculturally active ingredient and the emulsion concentrate of any one of claims 1 to 7 to a granulator to form granules, and drying the granules.

11. A method for forming a polymeric film on a substrate comprising applying the composition of claim 8 or claim 9 onto the substrate and removing any water therefrom.

12. A method for treating plants or soil comprising applying thereto a composition of claim 9 or granules obtained by the method of claim 10.

## Patentansprüche

1. Emulsionskonzentrat, umfassend:
a) 2 bis 90 Gew.-% eines Lösungsmittels, das aus der aus langkettigen Alkylpyrrolidonen, synthetischen oder natürlich vorkommenden hydrophoben Ölen und Gemischen davon ausgewählt ist;
b) 1 bis 60 Gew.-% eines wasserunlöslichen Pfropfpolymers aus N-Vinylpyrrolidon und einem α-Olefin, worin das N-Vinylpyrrolidon in einer Menge von zumindest 20 Gew.-% vorhanden ist; und
c) 1 bis 85 Gew.-% eines Tensids.

2. Konzentrat nach Anspruch 1, worin das Lösungsmittel ein langkettiges Alkylpyrrolidon der Formel: 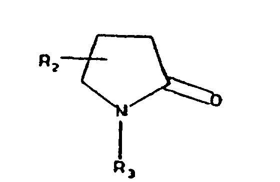 worin R₂ Wasserstoff oder Alkyl mit 6 bis 14 Kohlenstoffatomen ist und R₃ Alkyl mit 6 bis 14 Kohlenstoffatomen ist, mit der Maßgabe, dass zumindest eines von R₂ und R₃ zumindest 6 Kohlenstoffatome enthalten muss und die Summe der Kohlenstoffatome in R₂ und R₃ 14 nicht übersteigen kann, vorzugsweise R₂ Wasserstoff ist und R₃ C₉ oder C₁₂ ist, oder ein synthetisches oder natürlich vorkommendes Öl ist, das hydrophoben Charakter aufweist oder einen fraktionierten dispersiven Löslichkeitsparameter von über 70 % aufweist.

3. Emulsionskonzentrat nach Anspruch 1, das ein solches Lösungsmittel, das N-Octylpyrrolidon oder ein Rohöldestillat mit einem mittleren Siedepunkt von zumindest 120 °C ist, oder Gemische davon umfasst.

4. Konzentrat nach Anspruch 1, worin das wasserunlösliche Polymer ein Pfropfcopolymer aus N-Vinylpyrrolidon und einem α-Olefin ist, das 16 bis 20 Kohlenstoffatome enthält.

5. Konzentrat nach Anspruch 1, worin das wasserunlösliche Polymer ein Pfropfcopolymer aus N-Vinylpyrrolidon und einem C₂₀-α-Olefin in einem Gewichtsverhältnis von 50:50 ist.

6. Konzentrat nach Anspruch 1, worin das wasserunlösliche Polymer ein Pfropfcopolymer aus N-Vinylpyrrolidon und einem C₂₀-α-Olefin in einem Gewichtsverhältnis von 20:80 ist.

7. Konzentrat nach Anspruch 1, worin das Lösungsmittel aus N-Octylpyrrolidon mit 10 bis 20 Gew.-% oder 50 bis 70 Gew.-% des hydrophoben Öls oder Gemischen davon besteht, das wasserunlösliche Polymer in einer Menge von 10 bis 20 Gew.-% vorhanden ist und das Tensid in einer Menge von 5 bis 10 Gew.-% vorhanden ist.

8. Konzentrat nach einem der vorangegangenen Ansprüche, das weiters eine in der Landwirtschaft einsetzbare Chemikalie umfasst.

9. Zusammensetzung zur Anwendung auf landwirtschaftliche Erntepflanzen, umfassend eine Emulsion, die eine Zusammensetzung nach Anspruch 8 ist, die zumindest zweifach mit Wasser verdünnt ist.

10. Verfahren zur Herstellung eines in Wasser dispergierbaren Granulats aus einem in der Landwirtschaft einsetzbaren Wirkstoff, umfassend das Einfüllen eines in der Landwirtschaft einsetzbaren Wirkstoffs und eines Emulsionskonzentrats nach einem der Ansprüche 1 bis 7 in einen Granulator, um Granulat zu bilden, und das Trocknen des Granu lats.

11. Verfahren zum Ausbilden eines Polymerfilms auf einem Substrat, umfassend das Aufbringen einer Zusammensetzung nach Anspruch 8 oder Anspruch 9 auf das Substrat und das Entfernen von jeglichem Wasser daraus.

12. Verfahren zur Behandlung von Pflanzen oder Boden, umfassend das Aufbringen einer Zusammensetzung nach Anspruch 9 oder von Granulat, das nach einem Verfahren nach Anspruch 10 erhalten wurde, darauf.

## Revendications

1. Concentré en émulsion comprenant :
a) 2 à 90% en poids d'un solvant sélectionné dans le groupe consistant en alkyl pyrrolidones à chaîne longue, huiles hydrophobes synthétiques ou naturelles et leurs mélanges ;
b) 1 à 60% en poids d'un polymère greffé insoluble dans l'eau de N-vinyl pyrrolidone et d'une alpha-oléfine où la N-vinyl pyrrolidone est présente à raison d'au moins 20% sur une base pondérale ;
c) 1 à 85% en poids d'un agent tensioactif.

2. Concentré de la revendication 1, où le solvant est une alkyl pyrrolidone à chaîne longue ayant la formule 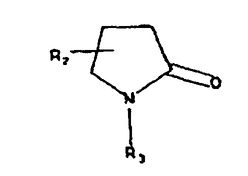 où R₂ est hydrogène ou alkyle ayant 6 à 14 atomes de carbone et R₃ est alkyle ayant 6 à 14 atomes de carbone, à condition qu'au moins l'un de R₂ ou R₃ doive contenir au moins 6 atomes de carbone, et la somme des atomes de carbone dans R₂ et R₃ ne peut dépasser 14, de préférence R₂ est hydrogène et R₃ est C₈ ou C₁₂, ou une huile synthétique ou naturelle ayant un caractère hydrophobe ou ayant un paramètre de solubilité dispersif fractionné de plus de 70%.

3. Concentré en émulsion de la revendication 1 comprenant un dit solvant qui est la N-octyl pyrrolidone ou un distillat d'huile brute ayant un point moyen d'ébullition d'au moins 120°C ou leurs mélanges.

4. Concentré de la revendication 1 où le polymère insoluble dans l'eau est un copolymère greffé de N-vinylpyrrolidone et d'une α-oléfine contenant 16 à 20 atomes de carbone.

5. Concentré de la revendication 1, où le polymère insoluble dans l'eau est un copolymère greffé de N-vinylpyrrolidone et d'une α-oléfine C₂₀ à un rapport pondéral de 50:50.

6. Concentré de la revendication 1, où le polymère insoluble dans l'eau est un copolymère greffé de N-vinylpyrrolidone et d'une α-oléfine C20 à un rapport pondéral de 20:80.

7. Concentré de la revendication 1, où le solvant est 10-20% en poids de N-octyl pyrrolidone ou bien 50-70% en poids de l'huile hydrophobe ou leurs mélanges, le polymère insoluble dans l'eau est présent en une quantité de 10 à 20% en poids et l'agent tensioactif est présent en une quantité de 5 à 10% en poids.

8. Concentré de l'une quelconque des revendications précédentes comprenant de plus un produit chimique actif pour l'agriculture.

9. Composition pour application à des récoltes agricoles comprenant une émulsion qui est une composition de la revendication 8 diluée au moins 2 fois avec de l'eau.

10. Méthode de production d'un granulé dispersible dans l'eau d'un ingrédient actif pour l'agriculture en chargeant un ingrédient actif pour l'agriculture et le concentré en émulsion de l'une quelconque des revendications 1 à 7 dans un granulateur pour former des granules et en séchant les granules.

11. Méthode pour former un film polymérique sur un substrat consistant à appliquer la composition de la revendication 8 ou de la revendication 9 sur le substrat et à en enlever toute eau.

12. Méthode pour le traitement de plantes ou du sol comprenant l'application à ceux-ci d'une composition de la revendication 9 ou des granules obtenus par la méthode de la revendication 10.
